# EUROPEAN PATENT SPECIFICATION

(11) **EP 1 592 968 B1**
(45) Date of publication and mention of the grant of the patent: **22.04.2009**
(21) Application number: 03776069.1
(22) Date of filing: 19.11.2003
(51) Int. Cl.: G01N 33/50

(54) **DETECTION OF CELLULAR OR DNA DAMAGE BASED UPON HR23 PROTEIN BINDING MOLECULES**
VERFAHREN ZUR ERKENNUNG VON ZELLEN ODER DNS SCHÄDEN DURCH HR23 PROTEIN BINDENDEN MOLEKÜLEN
DETECTION DE LESIONS CELLULAIRES OU DE L'ADN FONDEE SUR DES MOLECULES SE LIANT A UNE PROTEINE HR23

(30) Priority: 20.11.2002 EP 02079847
(43) Date of publication of application: 09.11.2005
(73) Proprietor: Erasmus Universiteit Rotterdam, 3015 GE Rotterdam (NL)
(72) Inventor: HOEIJMAKERS, Jan, Hendrik, Jozef, NL-2761 XW Zevenhuizen (NL); BERGINK, Steven,c/o Dep. of Molecular Cell Biology, 82152 Martinsried (DE); VAN DER HORST, Gijsbertus, Theodorus, Johannes, NL-3161 LK Rhoon (NL); VERMEULEN, Wim, NL-3333 VL Zwijndrecht (NL); NG,Mei,Yin St.Jude Children`s Research Hospital, Memphis, TN 38105-2794 (US)
(74) Representative: Winckels, Johannes Hubertus F.
(86) International application number: PCT/NL2003/000812
(87) International publication number: WO 2004/046683

(56) References cited:
- YAMAIZUMI MASARU ET AL: "U.v.-induced nuclear accumulation of p53 is evoked through DNA damage of actively transcribed genes independent of the cell cycle." ONCOGENE, vol. 9, no. 10, 1994, pages 2775-2784, XP009010086 ISSN: 0950-9232
- BATTY DAWN ET AL: "Stable binding of human XPC complex to irradiated DNA confers strong discrimination for damaged sites." JOURNAL OF MOLECULAR BIOLOGY, vol. 300, no. 2, 2000, pages 275-290, XP002239847 ISSN: 0022-2836
- HEY THOMAS ET AL: "The XPC-HR23B complex displays high affinity and specificity for damaged DNA in a true-equilibrium fluorescence assay." BIOCHEMISTRY, vol. 41, no. 21, 28 May 2002 (2002-05-28), pages 6583-6587, XP002239848 May 28, 2002 ISSN: 0006-2960
- SUGASAWA KAORU ET AL: "Xeroderma pigmentosum group C protein complex is the initiator of global genome nucleotide excision repair." MOLECULAR CELL, vol. 2, no. 2, August 1998 (1998-08), pages 223-232, XP002239849 ISSN: 1097-2765
- YOKOI MASAYUKI ET AL: "The xeroderma pigmentosum group C protein complex XPC-HR23B plays an important role in the recruitment of transcription factor IIH to damaged DNA." JOURNAL OF BIOLOGICAL CHEMISTRY, vol. 275, no. 13, 31 March 2000 (2000-03-31), pages 9870-9875, XP002239924 ISSN: 0021-9258
- NG JESSICA M Y ET AL: "Developmental defects and male sterility in mice lacking the ubiquitin-like DNA repair gene mHR23B." MOLECULAR AND CELLULAR BIOLOGY, vol. 22, no. 4, February 2002 (2002-02), pages 1233-1245, XP002239925 February, 2002 ISSN: 0270-7306
- SANTAGATI FABIO ET AL: "Different dynamics in nuclear entry of subunits of the repair/transcription factor TFIIH." NUCLEIC ACIDS RESEARCH, vol. 29, no. 7, 1 April 2001 (2001-04-01), pages 1574-1581, XP002239926 ISSN: 0305-1048

## Description

The integrity of a cellular organism is continuously challenged during its lifetime. Internal and external factors, such as toxic compounds and radiation, are a threat to the well-being of such organism. Potentially harmful factors comprise factors capable of distorting cellular processes such as the generation of vital biomolecules and/or degradation of such molecules, notably nucleic acids and proteins. For instance, inhibitors of RNA or protein synthesis, transport or turn-over compromise cellular function. Additionally, many internal and external factors are capable of damaging cellular components, such as DNA.

Preservation of an intact genome is of utmost importance to living cellular organisms. However, the integrity of nucleic acids such as DNA is continuously challenged. Cells must overcome endogenous (for instance metabolic) and exogenous (environmental) threats, as well as the intrinsic instability of chemical bonds in nucleic acid such as DNA itself (e.g. deamination and depurination). For instance, oxidative stress, ultraviolet (UV) light, ionizing radiation (such as X-rays), and numerous chemicals are capable of inducing a wide variety of lesions in DNA. An agent capable of inducing a DNA lesion is called a mutagen. A DNA lesion is defined herein as an alteration of DNA which involves a change in DNA sequence and/or a change in DNA structure. A DNA lesion can for instance comprise a DNA (double) strand break, and/or an insertion/deletion of at least one nucleotide.

A DNA lesion can affect cellular processes and can have severe consequences for the well-being of an organism. Direct effects of DNA lesions at the cellular level comprise inhibition of vital processes like transcription and replication, triggering cell cycle arrest. Accumulation of lesions in DNA above certain thresholds can lead to permanent alterations in the genetic code, replicative senescence and/or to (programmed) cell death. Permanent alterations in the genetic code can for instance cause changes in metabolic processes, inborn defects and/or overall functional decline contributing to (premature) aging. Mutations, specifically in proto-oncogenes and tumor suppressor genes, are responsible for tumor initiation and subsequent progression of the multistep process of carcinogenesis. Replicative senescence and cell death can enhance the process of aging.

In the art, potential mutagens are often tested with the widely used Ames test. This test is based upon reversion of mutations in a histidine (*his*) operon in the bacterium *Salmonella typhimurium.* The *his* operon encodes enzymes required for the biosynthesis of the amino acid histidine. Strains with mutations in the *his* operon are histidine auxotrophs: they are unable to grow without added histidine. Revertants that restore the *his*⁺ phenotype will grow on minimal medium plates without histidine.

In the Ames test the *his*⁻ mutants are mixed with a potential mutagen and then plated on minimal medium with a very small amount of histidine. The concentration of histidine used is limiting, so after the cells go through several cell divisions the histidine is used up and the auxotrophs stop growing. However, if the potential mutagen induces *his⁺* revertants during the initial few cell divisions, then each of the resulting revertants will continue to divide and form a colony. The number of colonies produced is proportional to how efficiently a mutagen reverts the original *his*⁻ mutation.

A disadvantage of the Ames test is that it is unable to detect mutagenic agents that are activated by the eukaryotic (organ or tissue-specific) cellular metabolism (such as the class of p450 enzymes). Although preincubation of the agent to be tested with cellular extracts may partly overcome this limitation the assay is still unreliable as it utilizes a bacterium to predict effects in a very different organism, such as a mammal, and/or in specific organs or tissues. Next to entire classes of false negative outcomes also a significant number of false positive results have been obtained with the heterologous procaryotic system. Moreover the test detects only mutagenic compounds but does not detect agents that have mainly a cytotoxic effect or induces deletions, or other chromosomal aberrations. Finally, the Ames test takes overnight incubation until the result is obtained. This is due to the fact that bacterial growth needs to be awaited.

It is an aim of the present invention to provide a novel test for detecting cells with RNA lesions. It is a further aim of the invention to provide a novel test for detecting agents that are harmful to eukaryotic organisms. More specifically, it is an aim of the present invention to provide a novel test for detecting agents that are mutagenic and/or cytotoxic for eukaryotic cells, and to provide a novel test for detecting agents capable of at least in part inhibiting proteolysis. It is a further object of the invention to provide a method for determining whether a cell has an impaired DNA damage repair mechanism.

The invention uses a method for detecting a DNA lesion in a eukaryotic cell, comprising determining whether a HR23 protein binding molecule accumulates within said cell. The invention provides a method for determining whether an agent is capable of inducing a DNA lesion that is substrate for global genome nucleotide excision repair in a eukaryotic cell, comprising:
- exposing at least one eukaryotic cell to said agent, and
- determining whether an overall intranuclear accumulation of xeroderma pigmentosum group C protein, or a functional part, derivative and/or analogue thereof which has the same kind of properties as XPC, occurs within said cell. It is determined whether said xeroderma pigmentosum group C protein or functional part, derivative and/or analogue thereof accumulates in the nucleus of said cell.

Xeroderma pigmentosum group C (XPC) protein is involved in a DNA repair mechanism called nucleotide excision repair (NER). NER primarily focuses on helix-distorting injuries, including UV-induced cyclobutane pyrimidine dimers (CPD) and pyrimidine (6-4) pyrimidone photoproducts (6-4PP), as well as numerous chemical DNA adducts (Friedberg et al., 1995). Inherited defects in NER are the cause of several severe diseases, such as the cancer-prone syndrome Xeroderma pigmentosum (XP). Patients are characterized by extreme sun sensitivity, sun-induced pigmentation anomalies, and a >2000-ford predisposition to UV-induced skin cancer. Moreover, an impaired DNA repair mechanism such as NER, or BER (described below) is involved in alteration of cells and their response to genotoxic agents.

NER entails a multi-step reaction and requires the coordinated action of ∼30 proteins implicated in damage detection, helix opening, lesion verification, dual incision of the damaged strand bracketing the injury, removal of the 26-30 base damage-containing oligonucleotide, gap-filling RNA synthesis and libation (Hoeijmakera, 2001). Two NER subpathways exist: global genome NER (GG-NER), operating genome wide and transcription-coupled repair (TCR), focusing on transcription-blocking lesions in the transcribed strand of active genes (Hanawalt, 2000). Most XP genes are implicated in both NER subpathways, but XPC-deficient cells are unique in being selectively deficient in GG-MER.

XPC is described in various publications.

Batty et al (J.Mol.Biol. (2000) 300, 275-290) show that XPC binds HR23B when there is a DNA lesion. An *in vitro* reconstituted nucleotide excision repair (NER) system was used which contained purified proteins and a defined cisplatin adduct. It was found that XPC activity in nucleotide excision repair was stimulated by addition of HR23B.

Hey et al (Biochemistry (2002) 41, 6583-6587) have performed a quantitative study on the binding of XPC to damaged DNA using fluorescence anisotropy measurements. It was shown that an XPC-HR23B complex discriminates between damaged and undamaged DNA sites with high specificity.

Sugasawa et al (Molecular Cell (1998) Vol. 2, 223-232) identified XPC-HR23B as the earliest damage detector to initiate NER. It was shown that XPC-HR23B binds to a variety of DNA lesions. The author suggest a two-step mechanism of damage recognition involving damage detection by XPC-HR23B followed by damaged verification by XPA.

Santagati et al (Nucleic Acids Research (2001) Vol. 29, No. 7, 1574-1581) investigated the subcellular localization of four subunits of the TFIIH complex, XPB, XPD, p62 and p44 and of XPC. It was shown that XPC, XPB, p62 and p44 proteins are located to the nucleus in response to UV light induced DNA damage.

3-Methyladenine DNA glycosylase (MAG) is involved in a DNA repair mechanism called base excision repair (BER). BER corrects base alterations induced by endogenous and/or exogenous oxidative events, ionizing radiation and small alkylating agents. Examples of potentially mutagenic BER-lesions are 8-oxoguanine, O⁶-methylguanine, deaminated methylated cytosine and thymine glycol. Base excision repair is initiated by MAG and several other glycosylase. Accumulating evidence implicates unrepaired BER lesions in the aging of somatic cells.

According to the present invention, a DNA lesion in a eukaryotic cell results in accumulation of a HR23 protein binding molecule within said cell. For instance, a NER sensitive RNA lesion results in rapid accumulation, of XPC of a eukaryotic cell, especially in the nucleus. Accumulation of a HR23 protein binding molecule, such as XPC is at least in part due to the fact that said HR23 protein binding molecule is stabilized by the chaperone proteins HR23A and/or HR23B if a DNA lesion is present. Both chaperon proteins are normally present in eukaryotic cells. In terms of the invention, a "HR23 protein" is defined as a HR23A or HR28B protein. It has been shown by the present inventors that only one of them suffices for stabilizing XPC. If no NER sensitive DNA lesion is present, XPC is rapidly degraded. A complex comprising HR23 and a HR23 protein binding molecule is involved in DNA repair. For instance, a complex comprising XPC and HR23 performs a damage-sensing step within GG-NER, triggering subsequent association of the New-involved proteins TFIIH, XPG, XPA, RPA, and ERCC1/XPF. Accumulation of XPC, or a functional part, derivative and/or analogue thereof, is therefore indicative for the presence of a DNA lesion that is substrate for GG-NER.

HR23A and HR23B are homologues of the *Saccharomyces cerevisiae* gene RAD23. The present inventors have cloned the two human homologues of RAD23, designated hHR23A and hHR23B. HR23 proteins contain a ubiquitin-like (Ub1) N-terminus and two ubiquitin-associated (UBA) domains pointing to multiple links with the ubiquitin system. The Ub1 domain of yeast RAD23 is important for UV-survival and for interaction with the 26S proteasome, whereas the UBA domains enable binding to ubiquitin. Until the present invention, the functional relationship between RAD23, RNA repair and the ubiquitin system was unclear.

Now it is found that HR23 protein binding proteins such as XPC accumulate in a eukaryotic cell upon a DNA lesion, a rapid and sensitive test is provided. A method of the invention is preferred over the widely used Ames test, because readout is based on the detection of accumulation of xeroderma pigmentosum group C protein, or a functional part, derivative and/or analogue there of which has the same kind of properties as XPC (for instance by way of fluorescence) and not on bacterial growth. A method of the invention can be performed quicker and allows for smaller quantities of a test compound as compared to the Ames test. Preferably, a method of the invention is performed within 3 to 6 hours. Moreover, a method of the invention provides more information about the effect of a test compounds upon a eukaryotic organism as compared to the Ames test. For instance, information is provided about transport of a test compound into a eukaryotic cell and into the nucleus of such cell, possible enzymatic modification of said test compound by a eukaryotic organism, and susceptibility of eukaryotic DNA. Moreover, a test of the invention provides information about the type of DNA damage induced by an agent, since different types of DNA lesions involve accumulation of specific HR23 protein binding molecules.

Potentially mutagenic or cytotoxic agents can be tested by exposing a eukaryotic cell to said agent, and determining whether a HR23 protein binding molecule Such as XPC or a functional part, derivative and/or analogue thereof, accumulates within said cell. In terms of the invention, by an agent capable of inducing a DNA lesion is meant an agent, such as for instance a compound or radiation, which is capable of inducing at least one DNA lesion. By an agent capable of inducing a RNA lesion that is substrate for GG-NER is meant an agent, such as for instance a compound or radiation, which is capable of inducing at least one DNA lesion that is normally recognized by a GG-NER system. By normally recognized is meant that in a (preferably naturally occurring) cell with functioning GG-NER system, such lesion is recognized by said GG-NER system. Of course, a capability of inducing DNA lesions often strongly depends on the dose of compound/radiation. Therefore, it is often suitable to test several amounts of test compound or several intensities of radiation with a method of the invention. However, if it is only questioned whether a specific dose of compound/radiation (for instance present in a new chemical compound, in a new procedure of purification, or in e.g. spoiled ground) is mutagenic, a test of the invention can be performed with only that dose.

A eukaryotic cell can be exposed to an agent in many different ways, which are known in the art. For instance, a potentially mutagenic compound can be administered to a culture comprising said eukaryotic cell. Alternatively, said compound can be administered to a non-human animal comprising said cell. Alternatively, a eukaryotic cell, for instance as part of a cell line or as part of a non-human animal, can be exposed to radiation. In one embodiment, several different doses of compound/radiation are used.

With a method of the invention, accumulation of an intact XPC protein in a eukaryotic nucleus can be determined. Alternatively, accumulation of a functional part, derivative and/or analogue of XPC can be tested. In a preferred embodiment, said XPC protein comprises a human XPC protein or a functional part, derivative and/or analogue thereof.

A functional part, derivative and/or analogue of an XPC protein can be provided to a eukaryotic cell with conventional methods known in the art, such as microinjection or transfection procedures. Such functional part, derivative and/or analogue can be provided to a eukaryotic cell by use of a nucleic acid encoding said functional part, derivative and/or analogue. Of course, said nucleic acid is preferably suitable for expression within said cell. As shown in the examples, it is also possible to provide a cell with a nucleic acid encoding a whole XPC protein. Said protein may be an endogenous XPC protein, or may be derived from a different kind of organism.

In a preferred embodiment said xeroderma pigmentosum group C protein, or a functional part, derivative and/or analogue thereof which has the same kind of properties as XPC is labelled to allow for easy detection. In one preferred embodiment, said label comprises green fluorescent protein (GFP) or luciferase. If GFP is used as a label, accumulation of xeroderma pigmentosum group C protein, or a functional part, derivative and/or analogue thereof which has the same kind of properties as XPC can be easily detected with a microscope or a fluorescent activated cell sorter (FACS) for easy and rapid quantitative read-out. A xeroderma pigmentosum group C protein, or a functional part, derivative and/or analogue thereof which has the same kind of properties as XPC can be labelled in many different ways and with many different labels known in the art. For instance, said label may be coupled to said molecule by way of a (flexible) linker. Said linker can be a peptide.

Said label can also be linked to said molecule in the form of a fusion protein, comprising both said molecule and said label. A nucleic acid can be constructed encoding such fusion protein by methods known in the art. Of course, the person skilled in the art can think of alternative ways of linking a label to XPC or to a functional part, derivative and/or analogue thereof.

Besides labeling of xeroderma pigmentosum group C protein, or a functional part, derivative and/or analogue thereof which has the same kind of properties as XPC accumulation of said molecule can also be detected in different ways that are known in the art. For instance, an antibody directed towards said molecule can be used. Binding of said antibody can be detected by staining said antibody, an affinity column may be used, etcetera. As another possibility, said molecule can be rendered radioactive.

A functional part of an XPC protein is defined as a part which has the same kind of properties as XPC in kind, not necessarily in amount. A functional part of XPC is for instance also capable of binding to HR28A and/or HR23B, and/or capable of entering the nucleus of a eukaryotic cell, optionally when bound to HR23A or HR23B. A functional derivative of an XPC protein is defined as a protein which has been altered such that the propertied of said molecule are essentially the same in kind, not necessarily in amount. A derivative can be provided in many ways, for instance through conservative amino acid substitution.

A person skilled in the art is well able to generate analogous compounds of an XPC protein. This can for instance be done through screening of a peptide library. Such analogue has essentially the same properties of an XPC protein in kind, not necessarily in amount.

As used herein, "an XPC proteins", or "XPC" are used interchangeable and can also mean a functional part, derivative and/or analogue of an XPC protein.

In a preferred embodiment, a method of the invention is provided wherein said eukaryotic cell is overexpressing HR23A and/or HR23B protein, or a functional part, derivative and/or analogue thereof. As shown in the examples, very good results are obtained if HR23A and/or HR23B is overexpressed. Overexpression of HR23A and/or HR23B can be performed in different ways. For instance, a nucleic acid encoding HR23A and/or HR23B can be constructed, preferably with a strong promoter. Such nucleic acid may comprise several copies of a gene encoding HR23A and/or HR23B. Overexpression of HR23A and/or HR23B can be induced by administration of said nucleic acid to a cell capable of expressing said nucleic acid. In one embodiment, said nucleic acid encodes human HR23A and/or human HR23B protein or functional part, derivative and/or analogue thereof. In another embodiment, said nucleic acid encodes murine HR23A and/or murine HR23B protein or functional part, derivative and/or analogue thereof.

Said nucleic acid may be expressed in a eukaryotic cell in addition to endogenously expressed HR23A and/or HR23B. Alternatively, said cell may be rendered deficient of endogenous HR23A and/or HR23B. Said nucleic acid preferably comprises a stronger promoter than the endogenous genes of HR23A and/or HR23B, enabling said cell to overexpress HR23A and/or HR23B.

In one embodiment a method of the invention is provided wherein said eukaryotic cell is a mammalian cell. Preferably, said cell is a murine cell, more preferably a mouse embryonic fibroblast. Said cell can be part of a cell line, such as a mouse embryonic fibroblast cell line. According to one embodiment, said cell is deficient in endogenous HR23A and/or HR23B protein. The cells of said cell line are also preferably deficient in endogenous HR23A and/or HR23B protein. Murine HR23A or HR23B protein is called mHR23A or mHR23B. A cell which is deficient for both endogenous HR23A and HR23B protein is preferably artificially provided with HR23A and/or HR23B. As disclosed in the examples, only one kind of HR23 protein is sufficient to preserve GG-NER activity. In another preferred embodiment, a method of the invention is provided wherein said XPC protein or functional part, derivative and/or analogue thereof comprises a human XPC protein or functional part, derivative and/or analogue thereof.

The invention also provides a method for screening of agents capable of at least in part inhibiting a cellular process that normally results in accumulation of xeroderma pigmentosum group C protein, or a functional part, derivative and/or analogue thereof which has the same kind of properties as XPC. For instance, in response to a DNA lesion, that is substrate for global genome nucleotide excision repair xeroderma pigmentosum group C protein, or a functional part, derivative and/or analogue thereof which has the same kind of properties as XPC accumulates within a cell. This involves many cellular processes, such as proteolysis, nucleo-cytoplasma shuttling, RNA synthesis, RNA processing, RNA transport, and/or RNA translation. If an agent is capable of inhibiting such process, accumulation of xeroderma pigmentosum group C protein, or a functional part, derivative and/or analogue thereof which has the same kind of properties as XPC will not, or less, occur when a DNA lesion is present. The invention therefore provides a method for determining whether an agent is capable of at least in part inhibiting a cellular process, such as proteolysis, nucleo-cytoplasma shuttling, RNA synthesis, RNA processing, RNA transport, and/or RNA translation, said process resulting in overall intranuclear accumulation of xeroderma pigmentosum group C protein, or a functional part, derivative and/or analogue thereof which has the same kind of properties as XPC, within a cell, comprising:
- exposing at least one eukaryotic cell to said agent, and
- determining whether an overall intranuclear accumulation of xeroderma pigmentosum group C protein, or a functional part, derivative and/or analogue thereof which has the same kind of properties as XPC, occurs within said cell. Preferably, said eukaryotic cell comprises a DNA, lesion.

Preferably, said proteolysis comprises proteasomal proteolysis. It has been shown by the present inventors that hXPC-GFP is mainly degraded via ubiquitin/proteasome-dependent proteolysis.

In one aspect the invention provides a mammalian cell which in deficient in endogenous HR23A protein or endogenous HR23B protein. Preferably, said cell is deficient in endogenous HR23A protein and endogenous HR23B protein. In one embodiment, said cell comprises a murine cell, preferably a mouse embryonic fibroblast. With a cell of the invention, a cell line can be generated. Such cell line is suitable for high throughput tests of compounds, for instance potentially mutagenic or cytotoxic compounds or compounds potentially capable of inhibiting proteolysis, with a method of the invention

A cell line comprising a cell of the invention is therefore also herewith provided.

As outlined in the examples, a non-human animal comprising a cell of the invention is also very suitable for testing and investigation purposes. The invention therefore also provides a non-human eukaryotic organism which is deficient in endogenous HR23A protein and/or endogenous HR23B protein. Since total HR23-deficiency is incompatible with animal life, an animal of the invention should comprise Her23, either one of the endogenous HR23A or HR23B proteins or functional part, derivative and/or analogue thereof, or an exogenous (such as human or murine) HR23 protein or functional part, derivative and/or analogue threat In one embodiment, said animal is provided with an exogenous controllable HR23 transgene. The invention also provides a non-human animal of the invention with compromised (endogenous) HR23 functions in a conditional fashion.

According to the invention, a HR23 protein binding molecule accumulates in a cell in response to a DNA lesion. For instance, XPC accumulates in a eukaryotic cell in response to a DNA lesion that is substrate for GG-NER. This applies to cells with a functioning GG-NER system. However, if a cell's GG-NER system is essentially impaired, XPC will not accumulate in the nucleus in response to DNA damage. Hence, accumulation of a HR23 protein binding molecule in a cell that is exposed to a DNA-affecting agent is indicative for an essentially functioning DNA repair system. The invention therefore provides a method for determining whether a cell has an at least partly impaired global genome nucleotide excision repair system, comprising:
- exposing said cell to an agent capable of inducing a DNA lesion, and
- determining whether an overall intranuclear accumulation of xeroderma pigmentosum group C protein, or a functional part, derivative and/or analogue thereof which has the same kind of properties as XPC, occurs within said cell.

Impaired NER and/or BER activity is associated with severe disorders, such as xeroderma pigmentosum (XP), cockayne syndrome. (CS) and trichothiodystrophy (TTD).

Xeroderma pigmentosum is due to a mutation in one of seven genes genes involved with NER (designated XPA to XPG). Parchment skin (Xeroderma) and freckles (pigmentosum) are the prominent cutaneous hallmarks of XP patients. These manifestations are strikingly restricted to sun-exposed areas of their skin. Typically, sun exposure of XP patients causes a progressive degenerative alteration of the skin and eyes, beginning as early as the age of 2 years. Furthermore, XP is associated with an elevated frequency (>1000-fold) of sunlight-induced skin cancers, which are also largely confined to sun-exposed areas like the face, neck, head and even the tip of the tongue. XP patients mainly develop basal cell or squamous cell carcinomas, seen in at least 45% of all XP patients, many of whom often have multiple primary neoplasms, and less frequently melanomas (5% ofpatients). The mean age of onset for skin neoplasms is 8 years, which is about 50 years earlier than in the general population. The main cause of death in XP individuals is neoplasia, which reduces the lifespan by approximately 30 years. XP patients also have a 10- to 20-fold increased risk of developing several types of internal cancers before the age of 20 years. Abnormalities in the immune system, detected in XP patients are likely to contribute to the development of (skin)tumors.

A fraction of XP patients (∼18%) displays progressive neurologic degeneration secondary to a loss of neurons. This feature seems to be related to the significance of the NER defect. For example XPC patients, who only have GG-NER defect, usually do not develop neurologic abnormalities, and if so, symptoms appear much later in life compared to TC-NER-defective XPD and completely NER-deficient XPA patients. A possible explanation for the onset of neurologic abnormalities in XP individuals is that defective DNA repair of endogenous, oxidative NER lesions in neurons triggers cell death. The genetic heterogeneity of XP patients is accompanied by heterogeneity in severity of the repair defect and of the consequent symptoms. The most severely affected patients are XPA, XPB, XPD and XPG individuals. The two most common forms of XP are XPA and XPC. The group of XPD patients is the most heterogeneous, with a level of residual repair synthesis between 15 and >50%. Furthermore, XPF patients are moderately UV-sensitive and show intermediate repair synthesis, indicative of mutations that lead to poor but not complete abolishment of NER. This could be due to the anticipated dual function of the ERCC1-XPF complex in NER and recombination repair. A null allele for ERCC1 or XPF and the consequential defect of crosslink repair are predicted to be incompatible with life. All XP patients of complementation groups A to G are defective in both NER subpathways, with the exception of XPC and XPE whose NER defect is limited to GG-NER. The susceptibility to sunburn of XPC patients is no different from normal individuals, indicating that TC-NER alone is sufficient to prevent this acute response to UV-exposure. XPC cells have a residual UDS level of 15-30% due to functional TC-NER and are therefore less sensitive to UV than XPA or XPD cells. Patients in the XP-variant group have mild to severe skin symptoms and usually display a normal functioning central nervous system. Unlike classical XP, XPV patients show a normal level of NER activity but lack the capacity to efficiently replicate damaged DNA leading to error prone replication and a hypermutable phenotype. This phenotype, together with the increased frequencies of genomic rearrangements observed in XPV cells may cause the elevated sun-induced carcinogenesis seen in these patients.

### II.5.2 Cockayne syndrome (CS)

CS is a very pleiotropic disorder characterized by cutaneous photosensitivity (with or without thin or dry skin and hair), several postnatal growth failure (cachectic dwarfism), mental retardation, and progressive neurologic dysfunction. CS cells are sensitive to a number of DNA-damaging agents (including UV) due to a defect in TC-NER. In contrast to patients suffering from the prototype NER-deficient disorder XP, CS individuals are not predisposed to skin cancer. Other common CS symptoms include sensorineural hearing loss, progressive ocular abnormalities (such as pigmentary retinopathy and/or cataracts), wizened bird-like faces, impaired sexual development, skeletal abnormalities (typically resulting in short stature), dental caries, kyphosis (hunchback), and premature osteoporosis (demineralisation). The progressive neurological degeneration has a very early onset in CS individuals (beginning around 2 years of age) and is caused by dysmyelination. The mean age of death in CS is 12.5 years and mainly secondary to pneumonia, which in turn could be due to the generally poor condition of the patients. Clearly, CS clinical symptoms are much more severe than the classical XP condition and go beyond photosensitivity. Photosensitivity and other XP-like features (such as pigmentation abnormalities and predisposition to skin cancer) can be attributed to the NER defect. However, the severe development and neurological manifestations of CS can not be explained by NER. The transcriptional engagement of CSA and CSB (analogous to XPB and XPD) suggests that transcription deficiency, perhaps induced by DNA damage, also contributes to the clinical pictures. In some cases, CS features are found in combination with XP, due to specific mutations in the XPB, XPD or XPG genes. Cells from CSA, CSB and XPG individuals with severe CS symptoms are slightly sensitive to ionizing radiation in addition to UV-light. It is hypothesized that inefficient TCR of oxidative lesions (e.g. thymine glycol) which block transcription underlies this ionizing radiation sensitivity although ionizing radiation is a poor inhibition of transcription in general. This indicates an additional role of CSA, CSB and XPG in coupling arrested transcription with both BER and NER, and suggests a general repair-transcription coupling deficiency as the major cause of the extensive variations in symptoms and severity of the CS phenotype. The developmental defects and the premature aging-related symptoms of CS can be attributed to the incomplete repair of endogenous oxidative damage, which in turn causes cellular malfunction and/or induction of apoptosis. The defective TRC in CS cells enhances their p53-dependent apoptotic response, contributing to the elimination of cells that potentially carry oncogenic mutations. This explains the lack of cancer predisposition in CS after UV-exposure. Numerous other CS-like patients have been identified, for example CAMFAK (for cataracts, microcephaly, failure to thrive, kyphoscoliosis) and COFS (cerebro-oculofacial syndrome), but these patients fail to exhibit pronounced photosensitivity in spite of the fact that cells of the patients display defective recovery of RNA synthesis, suggesting the possibility of a partial transcription defect without the accompanying TC-NER defect of CS.

### 5.3 Trichothiodystrophy (TTD)

TTD is caused by neurectodermal dysplasia which causes a collection of symptons referred to by the acronym PIBIDS: photosensitivity, ichthyosis, brittle hairs, intellectual impairment, decreased fertility, and short stature. Skeletal abnormalities are also frequently observed, including a peculiar bird-like face, a receding chin, and retardation of skeletal age. Moreover, axial osteosclerosis (abnormal hardening of the bone), peripheral osteoporosis and kyphosis have been reported. The striking ectodermal symptoms (brittle hair and dystrophic nails) are unique for TTD. However, the remainder of the clinical features are strikingly similar to CS, including the absence of cancer predisposition. The photosensitivity in TTD patients is due to a defect in NER caused by a mutation in one of three genes: XPB, XPD or TTDA. The NER defect in all but two of 20 studied UV-sensitive TTD families can be assigned to the XPD complementation group. Despite the NER defect, the pigmentation abnormalities are relatively mild compared to classical XP. The typical brittleness of TTD hair is caused by a substantial reduction in the content of hair-specific cysteine-rich matrix proteins that provide the hair shaft with its natural strength by crosslinking the keratin filaments. Growth retardation (cachectic dwarfism) in TTD patients is a very heterogeneous clinical symptom and - when severe - can be associated with death in early childhood. TTD, like CS, is considered to be a repair/transcription syndrome. Mutations in XPD may not only affect the NER function but also cripple transcription by TFIIH, accounting for the typical TTD and CS phenotypes. Consistent with this idea, all causative mutations in XPD have been found to be disease-specific. Recently, the phenotype of two unrelated TTDA patients was directly attributed to a limiting amount of TFIIH, probably secondary to a mutation in a gene determining the complex stability. A reduced TFIIH level has an effect on its repair function and also on its role in basal transcription.

A method of the invention is particularly suitable for determining whether an individual suffers from, or is at risk of suffering from, a disease associated with impaired DNA repair activity, such as XP, CS and/or TTD. With a method of the invention it can for instance be determined whether a cell from said individual has an at least partly impaired GG-NER system. If said cell appears to have an impaired DNA repair system, it is indicative for disease.

In one embodiment the invention therefore provides a method for determining whether an individuals suffers from, or is at risk of suffering from, a disease related to an at least partly impaired global genome nucleotide excision repair system, comprising:
- obtaining at least one cell from said individual,
- exposing said cell to an agent capable of inducing a DNA lesion, and
- determining whether an overall intranuclear accumulation of xeroderma pigmentosum group C protein, or a functional part, derivative and/or analogue thereof which has the same kind of properties as XPC, occurs in the nucleus of said cell.

In a preferred embodiment, said disease comprises xeroderma pigmentosum, cockayne syndrome, and/or trichothiodystrophy.

A kit of parts comprising a cell and/or a cell line of the invention is also herewith provided. Preferably, a kit of parts of the invention comprises an agent capable of inducing a RNA lesion, such as a lesion that is substrate for global genome nucleotide excision repair and/or base excision repair. More preferably, said kit of parts further comprises a detection system for detecting a change in level of a HR23 protein binding molecule. Most preferably, said kit of parts comprises a detection system for detecting a change in level of XPC, MAG, or a functional part, derivative and/or analogue thereof. A kit of parts of the invention is particularly suitable for performing a method of the invention.

The invention is further explained in the following examples. The examples only serve to more clarify the invention and do not limit the scope of the invention in any way.

### Examples

### Experimental Procedures

### Construction of mHR23A targeting vector

An Ola129 *mHR23A* targeting construct was generated by converting the BglII site in exon II of clone pG7M23Ag1 (containing a 4 kb genomic EcoRI fragment subcloned in pGEM7) into a ClaI site, which (due to a ClaI site in the polylinker) allowed deletion of sequences downstream of the BglII site in exon II (clone pG7M23Ag7). Next, the remaining EcoRI site was removed by filling-in the overhangs with Klenow, resulting in clone pG7M23Ag9. After changing the BstXI site into a SalI site, the 3 kb XhoI-SalI fragment was cloned into SalI digested pGEM5, resulting in clone pG5M23Ag17. Next, the 3' arm of the construct, consisting of a Klenow-blunted 1.5 kb SmaI-XbaI fragment starting at the SmaI site in exon VII, was inserted in the blunted NdeI site of pG5M23Ag17 (giving pG5M23Ag20), followed by insertion of a Neo marker cassette in antisense orientation in the ClaI site (giving pG5M23Ag24). Finally, the NotI-NsiI insert of pG5M23Ag24 was recloned into a pGEM-9Zf(-) based vector containing a 2.8 kb thymidine kinase (TK) marker cassette (giving pG5M23Ag30).

### ES cell culture and transfection

The Ola129-derived ES cell line E14 was electroporated with the *mnHR23A* targeting construct and cultured on dishes treated with gelatin as described previously (Ng et al., 2002). G418 (Geneticin, Gibco, final concentration 200 µg/ml) was added 24 hr after electroporation and cells were maintained under selection for 6-8 days. Genomic DNA from G418-resistent clones was digested with BamHI and subjected to Southern blot analysis using a 0.6 kb XbaI-RsaI fragment (3' external to the construct) as a probe. Targeted clones were subsequently screened with a Neo cDNA probe (ClaI fragment) to confirm proper homologous recombination in the 5' arm.

### Generation of the mHR23A^{-/-} and mHR23A^{-/-}/B^{-/}⁻ (DKO) mice and fibroblasts

Cells from two independent targeted clones with 40 chromosomes were injected into 3.5-day-old blastocysts isolated from pregnant C57BL/6 females (Ng et al., 2002). Male chimeric mice were mated with C57BL/6 females to obtain heterozygous animals. Germ line transmission was observed in the coat color of Fl offspring. Heterozygous males and females for *mHR23A* were interbred to generate *mHR23A*^{*+*/}*⁺, mHR23A*^{*+*/}*⁻,* and *mHR23A*^{*-*/}*⁻* mice. For the generation of double mutant *mHR23A*/*B* mice*,* male and female animals heterozygous for both *mHR23A* and *mHR23B* (Ng et al., 2002) were interbred. Genotyping was performed by Southern blot or PCR analysis of genomic DNA from tail biopsies of 10-14 day old pups.

Primary *mHR23A* MEFs (three independent lines per genotype) were isolated from day 13.5 embryos (E13.5) obtained from matings between *mHR23A*^{*+*/*-*}mice*.* Double mutant *mHR23A*/*B* MEFs were isolated from day 8.5 embryos (E8.5) derived from different crossings between *mHR23A*^{*+*/*-*}/*B*^{*+*/}*⁻* and *mHR23A*^{*-*/*-*}/*B*^{*+*/}*⁻* mice. Part of the embryo was used for genotyping and the remaining tissue was minced and immersed in a thin layer of F10/DMEM culture medium (Gibco BRL) supplemented with 15% fetal calf serum, 2mM glutamate, and 50 µg/ml penicillin and streptomycin. Spontaneously immortalized (established) cell lines were obtained by continuous subculturing of primary MEFs.

For the genotyping of E8.5 embryos the yolk sac was used as described (Gurtner et al., 1995). In short, the yolk sac was collected in 20 µl of water and immediately frozen in dry ice. Samples were heated for 5 min at 95°C and incubated with 1 µl of proteinase K (10 mg/ml) for 1 hr at 55°C. Proteinase K was heat-inactivated for 5 min at 95°C. PCR analysis was performed using the three-primer sets described below for 30 cycles (93°C, 1 min; 55°C, 1 min; 72°C, 90 sec) using *m.HR23A* and *mHR23B* primers.

Primer set 1: mHR23Ap1 (5'-atg-gga-ctt-ggg-cat-agg-tga-3'), mHR23Ap2 (5'-tct-tca-gcc-agg-cct-ctt-ac-3') and anti-sense neo (5'-atc-tgc-gtg-ttc-gaa-ttc-gcc-aat-g-3') giving 243 and 350 bp PCR fragments from the wildtype and targeted allele, respectively. Primer set 2: mHR23Bp1 (5'-gta-aag-gca-ttg-aaa-gag-aag-3'), mHR23Bp2 (5'-cta-cag-tct-tgt-ttc-tga-cag-3') and anti-sense pgk3 (5'-tag-ggg-agg-agt-aga-agg-tg-3') giving 202 and 600 bp PCR fragments from the wildtype and targeted allele, respectively.

### DNA repair assays and microneedle injection

UV sensitivity was determined as described (Ng et al., 2002). MEFs cultures were exposed to different doses of UV-C light (254 nm, Philips TUV lamp) and allowed to grow for another 3-5 days, before reaching confluence. The number of proliferating cells was estimated by scintillation counting of the radioactivity incorporated during a 3 hr pulse with [³H]thymidine (5 µCi/ml, specific activity (s.a.): 50 Ci/mmole; Amersham). Cell survival was expressed as the ratio of ³H incorporation in irradiated and non-irradiated cells.

UV-induced global genome repair was assayed using the UDS method as described (Vermeulen et al., 1994). Cells were exposed to 16 J/m² of 254 nm UV light and labeled with [methyl-³H]thymidine (10 µCi/ml, s.a.: 50 Ci/mmole). Repair capacity was quantified by grain counting after autoradiography.

RNA synthesis recovery after UV-irradiation was measured according to Ng et al. (2002). Cells were exposed to 10 J/m² of 254 nm UV light, allowed to recover for 16 hr, labeled with [5,6-³H]uridine (10 µCi/ml, s.a.: 50 Ci/mmole), and processed for autoradiography. The relative rate of RNA synthesis was expressed as the number of autoradiographic grains over the UV-exposed nuclei divided by the number of grains over the nuclei of non-irradiated cells on parallel slides.

Microneedle injection of control cells (C5RO) was performed as described previously (Vermeulen et al., 1994). After injection of at least 50 homopolykaryons cells were cultured for the desired time in normal culture medium before they were assayed for their repair capacity by means of UV-induced UDS.

### RNA and protein analysis

Total RNA was isolated from *mHR23A* MEFs using a RNeasy Mini Kit (Qiagen). 20 µg of total RNA was separated on a 0.9% agarose gel and transferred to Hybond-N+ membrane (Amersham Pharmacia Biotech). RNA blots were hybridized using *mHR23A* and β-*actin* ³²P-labeled cDNA probes.

Immunoblot analysis was performed on fibroblast extracts obtained by sonification [5x10⁶ cells in 300 µl phosphate-buffered saline (PBS)] or extraction. In the latter case, NP lysis buffer [25 mM Tris-HCl (pH 8.0), 1 mM EDTA, 10% glycerol, 0.01% Nonidet P-40, 1 mM dithiothreitol, 0.25 mM phenylmethylsulfonyl fluoride, and protease inhibitor mix (chymostatin, leupeptin, antipain, and pepstatin A)] was added to a monolayer of MEFs. After 30 min on ice the lysate was collected with a cell scraper and clarified by 2 times centrifugation at 4°C. NP lysis buffer containing 0.3 M NaCl was added to the cell pellet and homogenized by sonification.

SDS polyacrylamide gel electrophoresis was performed by loading 25-50 µg of total cellular protein per lane on 6-8% gels. Proteins were blotted to nitrocellulose membranes (Schleicher & Schuell) and probed with polyclonal antibodies recognizing human HR23A or XPC, or with monoclonal antibodies recognizing the HA epitope (HA.11, BAbCO) or p62 subunit of TFIIH (C39, kindly provided by Dr. J.M. Egly). Proteins were visualized using alkaline phosphatase-labeled goat anti-rabbit or peroxidase-conjugated goat anti-rabbit or goat anti-mouse secondary antibodies.

### Immunofluorescence labeling

Cells were grown on glass coverslips at 60-80% confluence. After washing twice with PBS, cells were fixed with 2% paraformaldehyde in PBS for 10 min at room temperature (RT) and permeabilized with 0.1% Triton X-100 in PBS for 2x 10 min at RT. After extensive washing (three times of 5 min each) with PBS⁺ (PBS supplemented with 0.15% glycine and 0.5% BSA) cells were incubated with affinity-purified primary antibodies in PBS⁺ in a moist chamber for 1½ hr at RT. After washing five times in PBS⁺, cells were incubated with the secondary antibodies for 1½ hr in PBS⁺ in a moist chamber at RT. Following 5 washes with PBS⁺ and once with PBS, coverslips were preserved with Vectashield^{™} Mounting Medium (Vector Laboratories) containing 4'-6-diamidino-2-phenylindole (DAPI, 1.5 µg/µl) to visualize the nuclei.

Primary antibodies used: affinity-purified, rabbit polyclonal anti-human XPC; rabbit polyclonal anti-human ERCC1; rabbit polyclonal anti-XPA (a kind gift from Dr. K. Tanaka); mouse monoclonal anti-p62 of TFIIH subunit (3C9, J.M. Egly, Illk-irch); and high affinity, rat monoclonal anti-HA (3F10, Boeringer). Secondary antibodies were: goat anti-rat and goat anti-rabbit Alexa 594-conjugated, and goat anti-rat and goat anti-rabbit Alexa 488-conjugated antibodies (Molecular probes); and goat anti-mouse Cy3-conjugated antibodies (Jackson ImmunoResearch Laboratories).

### Generation of XPC-GFP fusion cDNA construct and cotransfection studies

Full length *h*uman *XPC* cDNA (ScaI-Asp718I fragment) was cloned in EcoRI-Asp718I digested eukaryotic expression vector pEGFP-N3 (Clontech) containing a 3' histidine-hemaglutinine tag (generated by insertion of a doublestranded oligonucleotide in SspBI-NotI digested pEGFP-N3; kindly provided by D. Hoogstraten). For simplicity, the resulting tagged cDNA construct *hXPC-EGFP-His₆HA-N₃* is referred to as *hXPC-GFP*.

Full length cDNAs of the *hHR23B* (in a pSLM vector, Pharmacia biotech) and *hXPC-GFP* were cotransfected into *DKO* MEFs using puromycin as selectable marker. The transfection was performed using SuperFect Transfection Reagent (Qiagen) and puromycin was added 24 hr after transfection to a final concentration of 1 µg/ml, and the cells were maintained under selection for 20-40 days. Stable puromycin-resistant clones were isolated and integration of the cDNA construct was confirmed by DNA blotting (data not shown).

### Exposure of cells to DNA damaging agents

Cells stably expressing hXPC-GFP/hHR23B were rinsed with PBS, exposed to UV-C light (254 nm; Philips TUV lamp, dose as indicated in the text) and subsequently cultured at 37°C for various time periods (as indicated in the text). XPC was detected either by immunoblot analysis or by visualization in living cells using fluorescence microscopy. A similar approach was used to study the effect of N-acetoxy-2-acetylaminofluorene (NA-AAF, final concentration 50 or 100 µM), mitomycin C (MMC, Sigma, final concentration 1.2 or 2.4 µg/ml), ionizing radiation (y-rays from a ¹³⁷Cs source, single dose of 6 and 10 Gy), the proteasome inhibitor N-CBZ-LEU-LEU-LEU-AL (CBZ-LLL, Sigma, final concentration 5 or 10 µM), the transcription inhibitor 5,6-dichloro-1B-D-ribofuranosyl-benzimidazole (DRB, Sigma, final concentration of 100 µM, 2-3 hrs), the translation inhibitor cyclohexamide (CHX, Boehringer, final concentration 30, 50, and 100 µg/ml, 1-3 hrs,), heat shock (39.5 and 41°C, for 2-12 hrs), and the nuclear export inhibitor leptomycin B (LMB, Sigma, final concentration 10 ng/ml).

Local UV-irradiation was obtained by covering cells grown on glass coverslips with an isopore polycarbonate filter with pores of 5.0 µm diameter (Millipore, TMTP) during UV-irradiation (4 x 16 J/m² UV-C). Immediately after exposure, the filter was removed and medium was added back to the cells and culturing was continued. After various time periods (as indicated in the text), cells were processed for immunolabeling.

To identify cells in mixtures of control and mutant fibroblasts, cells were labeled with latex beads (diameter 0.79 µm, Polybead Carboxylate Microspheres, Polysciences) added to fibroblasts cultures 2 days prior to mixing of the cells. Cells were thoroughly washed in PBS (3x) before trypsinization to remove the non-incorporated beads and seeded in a 1:1 ratio on coverslips and cultured for 2 days.

### Heterokaryon nuclear-cytoplasmic shuttling assay

The shuttling assay using heterokaryons was performed as described (Borer et al., 1989). One day before cell fusion, *DKO* cells stably expressing hXPC-GFP/hHR23B and HeLa cells were seeded in a 1:1 ratio on coverslips. Six hours prior to fusion cells were irradiated with 10 J/m² UV-C or treated with 10 µM CBZ-LLL. Cell-fusion was induced (after washing with PBS) by treatment with 50% polyethylene glycol 6'000 in HANKS (Gibco) for 2 min followed by (3x) washing with PBS. Finally, cells were cultured in fresh medium either supplemented with or without leptomycin B (LMB, final concentration 10 ng/ml). Three to five hours after fusion cells were fixed with 2% paraformaldehyde and immunostained with rat monoclonal anti-HA (to monitor the XPC-GFP-His₆HA protein) and rabbit polyclonal anti-human ERCC1 (to distinguish human nuclei from mouse nuclei, since it specifically recognizes human ERCC1) and subsequently with appropriate secondary antibodies (see above).

### Light microscopy and image analysis

Immunofluorescent microscopy images were obtained with either a Leitz Aristoplan microscope equipped with epifluorescene optics and a PlanApo 63x/1,40 oil immersion lens or a Leica DMRBE microscope equipped with epifluorescene optics and a PL Fluotar 100x/1.30 oil immersion lens. For the detection of GFP-tagged proteins in the living cell, we have used an Olympus IX70 microscope equipped with epifluorescence optics and Olympus PlanApo 60x/1.40 oil immersion lens. GFP images were obtained after excitation with 455-490 and long pass emission filter (>510 nm). Cy-3 images were obtained after excitation with 515-560 and long pass emission filter (580 nm).

### Results

### Generation of mHR23A-deficient mice and cells

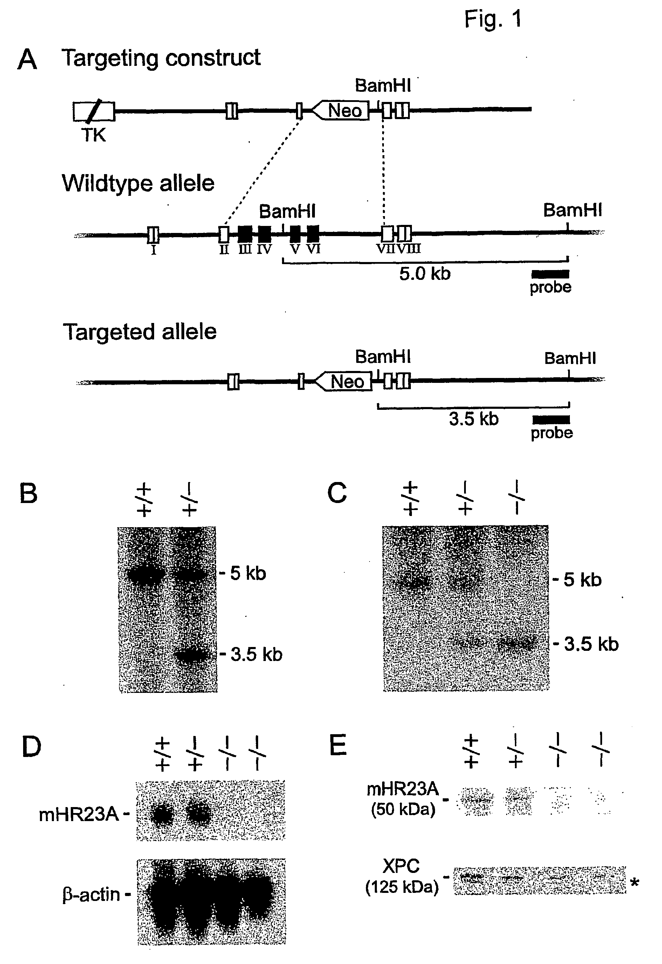

To generate a mouse model for mHR23A, we created a targeting construct in which exons III to VI, and part of exon II and VII (encoding residues 55 to 288 of the mHR23A protein) were replaced by the neomycin resistance marker. Gene targeting creates a *mHR23A* allele encoding a severely truncated protein in which >85% of the coding sequence is deleted (even truncating the UbL domain) and thus can be considered a null-allele (Figure 1A). Two correctly targeted clones (obtained at a frequency of 16%, Figure 1B) were used for blastocyst injections. Heterozygous offspring from matings between germ line chimeric males and C57BL/6 female mice was intercrossed to generate homozygous mutant *mHR23A* animals (Figure 1C), as well as day 13.5 embryos (E13.5) for isolation of mouse embryonic fibroblasts (MEFs). Neither the *mHR23A* mRNA, nor the 50 kDa mHR23A protein could be detected in *mHR23A*^{*-*/}*⁻* MEFs (Figure 1D and 1E). The two independent mouse lines were biochemically and phenotypically indistinguishable for all parameters tested.

### mHR23A^{-/-} animals and MEFs are NER proficient

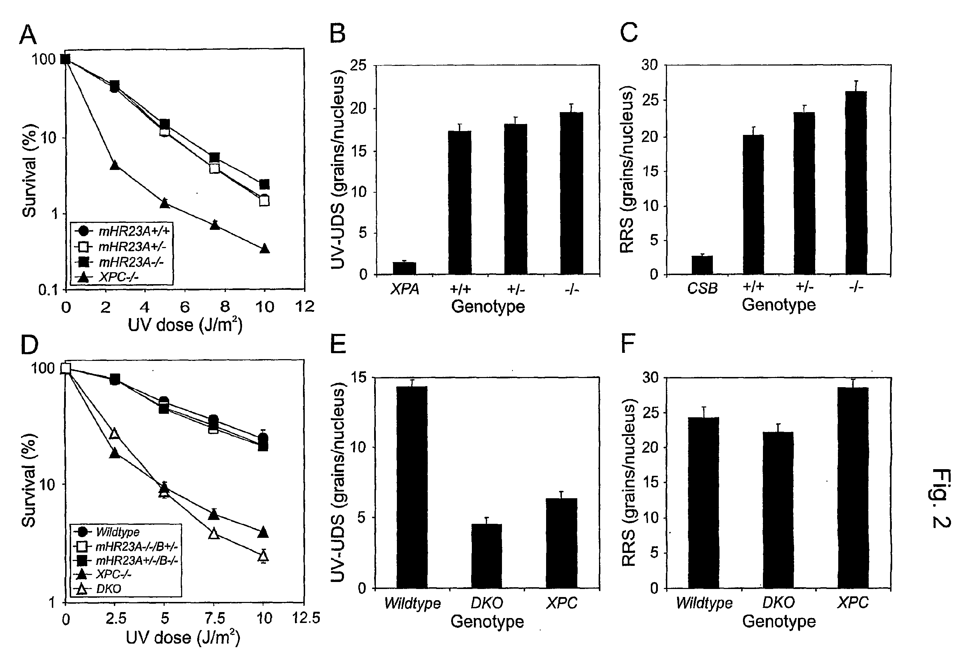

We assessed key repair parameters in *mHR23A*^{*-*/}*⁻* MEFs. As shown in Figure 2A-C, UV- survival, UV-induced unscheduled DNA synthesis (UDS), and RNA synthesis recovery after UV-exposure were all in the wildtype range, indicating that global as well as transcription-coupled NER are unaffected, mimicking the situation in a *mHR23B* mutant (Ng et al., 2002). These data show that mHR23A and mHR23B are functionally redundant for NER *in vivo.*

In striking contrast to *mHR23B*^{*-*/}*⁻* animals, *mHR23A*^{*-*/}*⁻* were born with Mendelian frequency and appeared indistinguishable from wildtype and heterozygous littermates for all parameters tested (including morphology, main pathology, and growth rate up to 16 months). *mHR23A*^{*-*/}*⁻* male and female mice were fertile, and their mating activity and litter size were normal. Apparently, mHR23A is not essential for mouse development and mHR23B can compensate for any additional functions of mHR23A.

### Total mHR23-deficiency is incompatible with animal life

In order to investigate the effect of a total *mHR23*-deficiency, we tried to generate *mHR23A*^{*-*/}*⁻lB*^{*-*/}*⁻* animals (hereafter referred to as: '*DKO*' for double knockout) and to obtain corresponding MEFs, by double heterozygous matings. Remarkably, out of 427 newborns analyzed no *DKOs* were found (Table 1). This shows that inactivation of *mHR23A* aggravates the severe developmental defects caused by a *mHR23B*-deficiency (Ng et al., 2002) to a level incompatible with life. Whereas we obtained phenotypically normal *mHR23A*^{*-*/*-*}/*B*^{+/-} mutant mice at Mendelian ratios (71/427 found and 83/427 expected), surprisingly *mHR23A*^{*+*/}*⁻lB*^{*-*/}*⁻* animals were not born (0/427). However, we were able to isolate E13.5 *mHR23A*^{*+*/*-*}/*B*^{*-*/}*⁻* mutant MEFs, although they showed poor growth. Apparently, loss of even one allele of *mHR23A* in a complete *mHR23B* null-background causes lethality in embryogenesis.

To investigate embryonic lethality caused by a complete *mHR23*-deficiency we isolated embryos at various stages of development. No *DKO* embryos were present at day 13.5 and 10.5, but growth-retarded *mHR23*-deficient embryos were observed at day 8.5. Importantly, three *DKO* MEF-lines were isolated from E8.5 embryos (3/43, see Table 1). Compared to wildtype and double heterozygous mutant MEFs, these cells displayed reduced rates of proliferation, which resulted in the loss of two lines. Nevertheless, we succeeded in establishing one *DKO* cell line after 30 weeks culturing, which permitted functional characterization of a total *mHR23*-deficiency.

### Total mHR23-deficient cells show an XPC-like repair phenotype

Cell survival experiments revealed that *DKO* MEFs are remarkably similar to the unique NER phenotype of *XPC*^{-/-} cells in terms of UV survival (Figure 2D), deficiency of ITV-induced UDS and proficiency of RNA synthesis recovery after UV-exposure (Figure 2E and F). In contrast, MEFs retaining only one *mHR23A* or *mHR23B* allele were NER competent (Figure 2D). Apparently, one out of four *mHR23* copies is sufficient for normal NER activity.

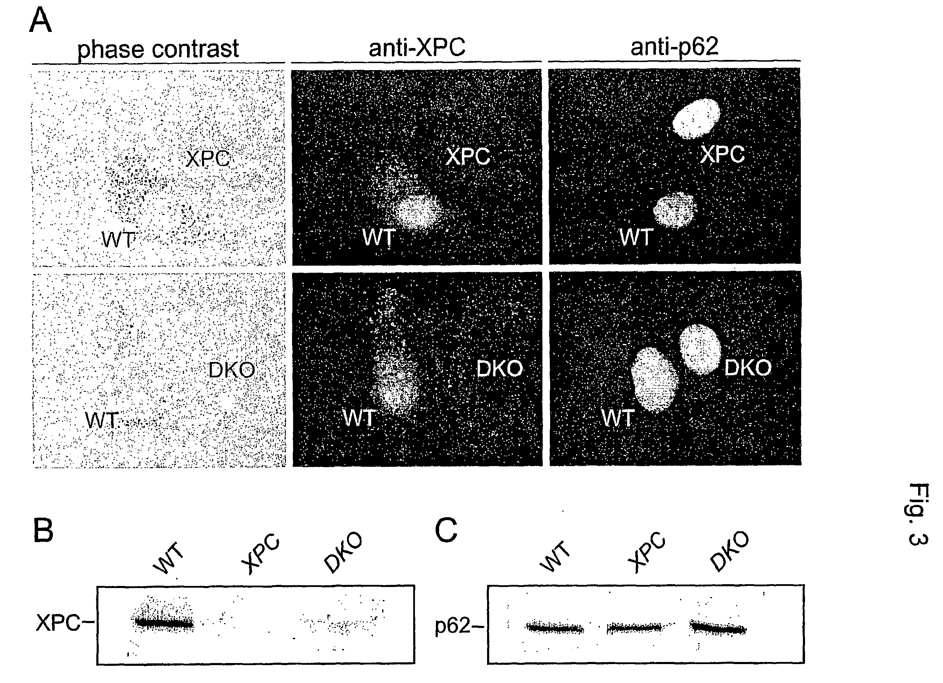

We have examined the status of the XPC protein in the *DKO* MEFs. Interestingly, steady-state levels of XPC appeared strongly reduced in *DKO* MEFs compared to wildtype and *mHR23A*^{*-*/}*⁻* cells (Figure 1E), as shown by comparative immunofluorescence (Figure 3A) and immunoblot analysis of cell extracts (Figure 3B). Thus, in the absence of both mouse RAD23 proteins XPC is unstable.

### hHR23B and hXPC-GFP rescue the UV-sensitivity of DKO cells

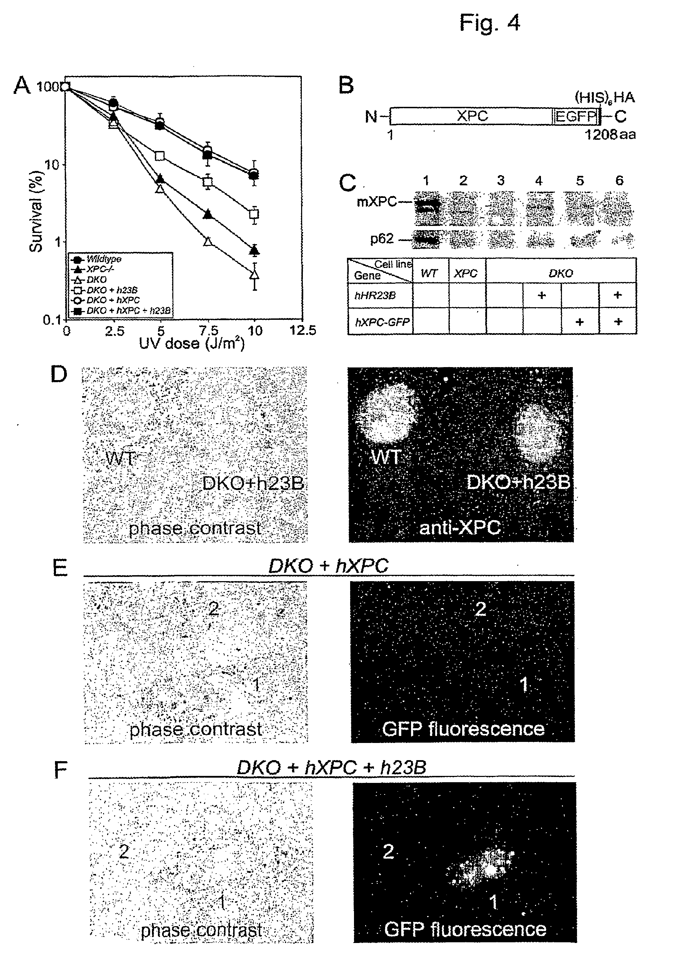

To provide direct evidence that the *XPC*-like phenotype of *DKO* cells is specifically caused by the *mHR23* defect, we stably transfected (*human*) *hHR23B* cDNA into *DKO* MEFs. The UV-sensitivity of *DKO* cells hHR23B was only partly rescued, perhaps due to human-mouse differences (Figure 4A). Importantly, expression of hHR23B induced an increase in the total amount of endogenous (*m*ouse) mXPC, as shown by both immunoblot (Figure 4C, lane 4) and immunofluorescence analysis (Figure 4D).

Subsequently, we generated double mutant MEFs that stably express (*h*uman) hXPC, tagged with GFP (and additional His6 and HA tags) (Figure 4B), to allow direct observation in living cells. Functionality of the hXPC-GFP was demonstrated after microinjection and transfection of the cDNA construct in XPC-deficient cells (data not shown). Although hXPC-GFP was undetectable by fluorescence microscopy (Figure 4E), stable transformants (verified for the presence of *hXPC-GFP* cDNA by DNA blotting) had largely regained wildtype UV-resistance (Figure 4A) indicating that the repair defect was rescued. Introduction of hXPC-GFP appeared to restore endogenous mXPC levels as shown by immunoblot (Figure 4C, lane 5) and immunofluorescence analysis (not shown). Apparently, hXPC-GFP has a trans-effect on mXPC stability.

To investigate the stabilizing effect of mHR23B on XPC, we cotransfected *hHR23B* with *hXPC-GFP* cDNA into *DKO* cells. Stably transfected clones exhibited wildtype UV-resistance (Figure 4A) and normalized levels of endogenous mXPC (Figure 4C, lane 6, and not shown). In contrast to MEFs expressing only hXPC-GFP, a small fraction (<10%) of the double cotransfected cells displayed green fluorescent nuclei (Fig 4F). This is due to a level of hXPC-GFP expression below the detection limit since immunofluorescence using anti-HA monoclonals revealed that the majority of the cells expressed the tagged transgene (data not shown). These data show that the cotransfected hHR23B acts as a stabilizing factor for both hXPC-GFP and endogenous mXPC.

### DNA damage causes accumulation of hXPC-GFP

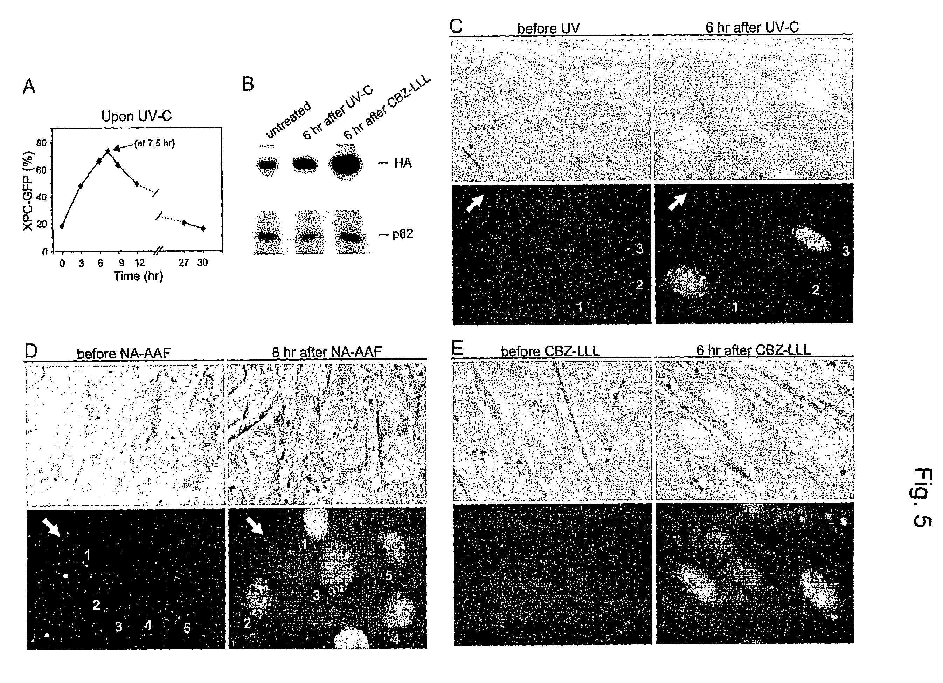

The hXPC-GFP/hHR23B *DKO* cell line provided a convenient tool to monitor the effect of DNA damage on XPC steady-state level and mobility in living cells. Interestingly, W-irradiation (5 and 10 J/m²) strongly increased the percentage of green cells and the intensity of the GFP signal. Kinetic analysis upon UV-exposure revealed a time-dependent reversible accumulation of XPC-GFP in the majority of the cells (Figure 5A); this was further illustrated by monitoring individual cells in time after UV-irradiation (Figure 5C). In addition, these findings were corroborated by immunoblotting of whole cell extracts using antibodies against the HA epitope attached to the GFP tag (Figure 5B, lane 2) and anti-HA immunocytochemistry (not shown). Since this phenomenon was specific for *DKO* cells transfected with hXPC-GFP/hHR23B these results show that XPC levels are responsive to UV in an HR23-dependent fashion.

To investigate whether XPC accumulation is specific for NER-type DNA damage or just stress-related, cells were exposed to different kinds of genotoxic agents. N-acetoxy-2-acetylaminofluorene (NA-AAF, 50 and 100 µM), which induces bulky adducts processed by NER, elicited a very potent Us-like response in all cells within 6 to 8 hrs (Figure 5D). In contrast, Y-rays (6 and 10 Gy) and mitomycin C (MMC, 1.2 and 2.4 µg/ml), inducing mainly strand breaks and interstrand crosslinks respectively (which are dealt with by other repair pathways) failed to provoke detectable XPC accumulation. Also heat shock (41°C, analyzed for up to 12 hrs) failed to boost fluorescence. The possibility that UV and NA-AAF evoke a general accumulation of protein was ruled out since cells expressing GFP alone do not exhibit a significant increase in fluorescence after genotoxic insults. This shows that lesions specifically recognized by the NER pathway enhance the level of HR23-dependent hXPC-GFP.

One of the direct consequences of UV- and NA-AAF-induced DNA damage is a temporary block of transcription. To investigate whether hXPC-GFP accumulation requires transcription or is induced by a DNA damage independent blockage of transcription, mRNA synthesis in *DKO* cells expressing hXPC-GFP/hHR23B was reversibly arrested by incubation with 5,6-dichloro-1β-D-ribofuranosyl-benzimidazole (DRB, 100 µM). No induction of XPC-GFP fluorescence was observed: in stead preincubation with DRB (2-3 hrs) prior to UV treatment prevented UV-induced XPC-GFP accumulation (data not shown). Consistent with this result, no enhanced XPC fluorescence was found in cells treated with the translational inhibitor cyclohexamide (30 and 50 µg/ml), demonstrating the requirement for *de novo* RNA and protein synthesis. In non-challenged conditions the steady-state level of XPC remains low.

### hXPC-GFP is degraded via ubiquitinlproteasome-dependent proteolysis

To further examine the HR23-dependent XPC stabilization, *DKO* cells expressing hXPC-GFP/hHR23B were incubated with the proteasomal proteolysis inhibitor N-CBZ-LEU-LEU-LEU-AL (CBZ-LLL, 5 and 10 µM) (Wiertz et al., 1996). Similar to UV-irradiation and NA-AAF, all cells displayed a striking XPC-GFP accumulation in time (Figure 5E), which was reversible upon drug removal (not shown). Both, immunoblot analysis (Figure 5B, lane 3) and immunocytochemistry, using anti-HA antibodies (not shown) confirmed the above observations. These findings show that degradation of XPC-GFP occurs via ubiquitin/proteasome-dependent proteolysis and that an agent capable of at least in part inhibiting proteolysis can be detected by determining whether XPC accumulates in a cell.

### Application of local UV damage to hXPC-GFP expressing cells

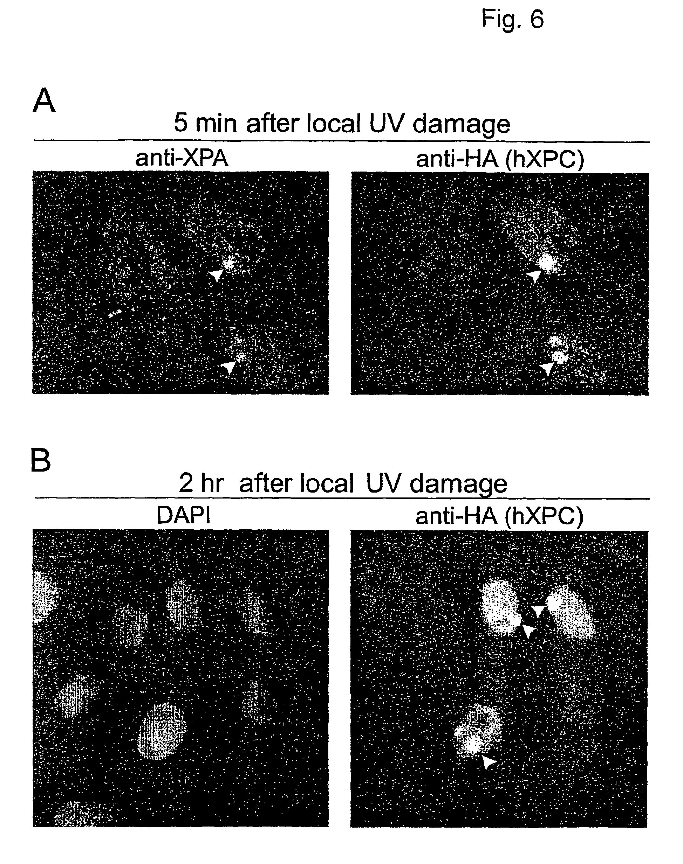

To explore the mechanism by which hXPC-GFP is stabilized, we employed a recently developed method for induction of DNA damage in a restricted part of the nucleus. For this purpose, a monolayer of *DKO* cells expressing hXPC-GFP/hHR23B was covered with an UV-light shielding isopore polycarbonate filter (pore diameter ~5 µm). Upon UV-irradiation, only at the position of pores UV-damage is induced, as detected with antibodies that specifically recognize CPD and 6-4PP lesions. These locations attract all NER proteins tested thus far. Cells were fixed at different time points after UV to allow simultaneous immunostaining with antibodies against various proteins and GFP fluorescence microscopy (Figure 6). Non-irradiated nuclei and non-damaged regions within partly irradiated nuclei serve as internal controls. Very rapidly (<2 minutes) after UV-exposure, GFP fluorescence and anti-HA immunostaining revealed high local accrual of hXPC-GFP(His₆HA) in part of the nuclei, which colocalized with XPA (Figure 6A) and the p62 subunit of TFIIH (not shown). These findings demonstrate that in living cells the GFP-tagged XPC protein translocates very rapidly to sites containing UV lesions.

If XPC stabilization only occurs when bound to the damage, an increase in fluorescent signal selectively at the damaged sites would be expected. On the other hand, with an (additional) overall stabilization of hXPC, it is expected that in time a concomitant increase of fluorescence over the entire nucleus (in addition to the damaged area) will be observed in comparison to non-damaged nuclei. The increase of hXPC-GFP (Figure 6A) initially occurs only at the locally damaged sites, but after two hours also in the remainder of locally damaged nuclei, a clearly higher signal is noted when compared to non-exposed nuclei in the vicinity (Figure 6B). These findings demonstrate an overall intranuclear stabilization of hXPC-GFP triggered by binding to lesions.

### High levels of XPC mediate a transient enhancement of DNA repair

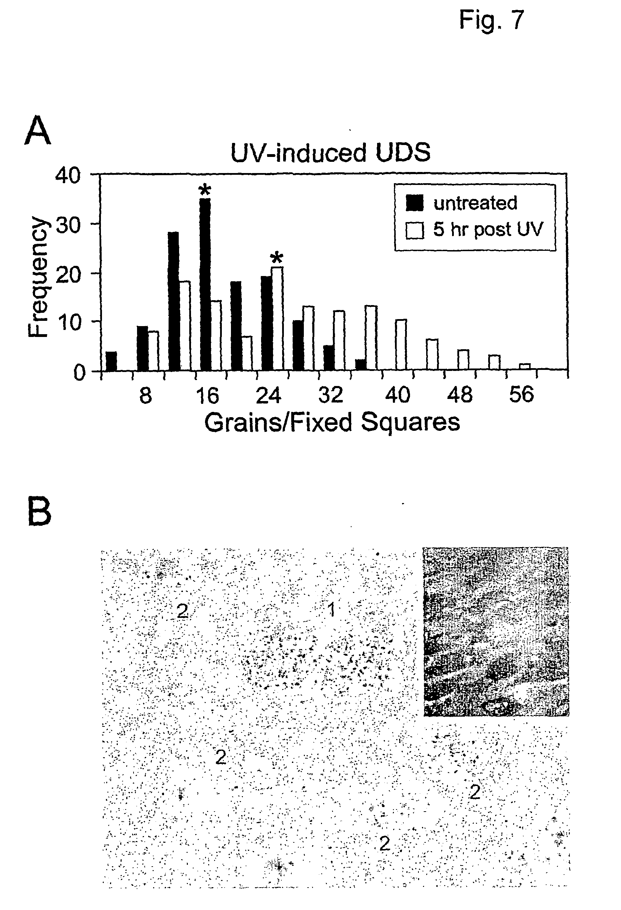

To investigate the biological consequence of DNA damage-induced stabilization of XPC, we tested the DNA repair capacity (UV-induced UDS) in *DKO* cells expressing XPC-GFP/hHR23B pre-challenged with UV-light. 5 hr post UV-irradiation (10 J/m²) the mean UDS level (as determined by 1 hr ³H-thymidine pulse-labeling immediately after a dose of 16 J/m²) was 1.5-fold increased compared to cells assayed in parallel that were not pre-irradiated (Figure 7A). UV-induced XPC-GFP accumulation was confirmed microscopically (data not shown) just prior the UDS assay. The increase in UDS is not derived from the additional effect of NER still dealing with lesions remaining of the first UV-dose, since in a separate UDS experiment without the second UV-irradiation no significant UDS was observed (not shown). These data demonstrate that UV-induced accumulation of XPC-GFP causes a concomitant increase in GG-NER. Enhanced repair by increased levels of XPC was confirmed by microinjection of *XPC-GFP* cDNA into homopolykaryons of wildtype human fibroblasts. Microinjected cells expressing XPC-GFP (Figure 7B, top right panel) exhibit a higher UDS compared to neighboring, non-injected monokaryons (Figure 7B). In contrast, when a cocktail of *XPC-GFP* and *hHR23B* was injected UDS in the majority of the cells was significantly lower and injection of this cocktail appeared highly toxic (data not shown). These data demonstrate that large amounts of stabilized XPC (as a result of overexpressed hHR23B) can reduce cell viability.

### Sequestration of XPC in the nucleus caused a reduced proteolysis

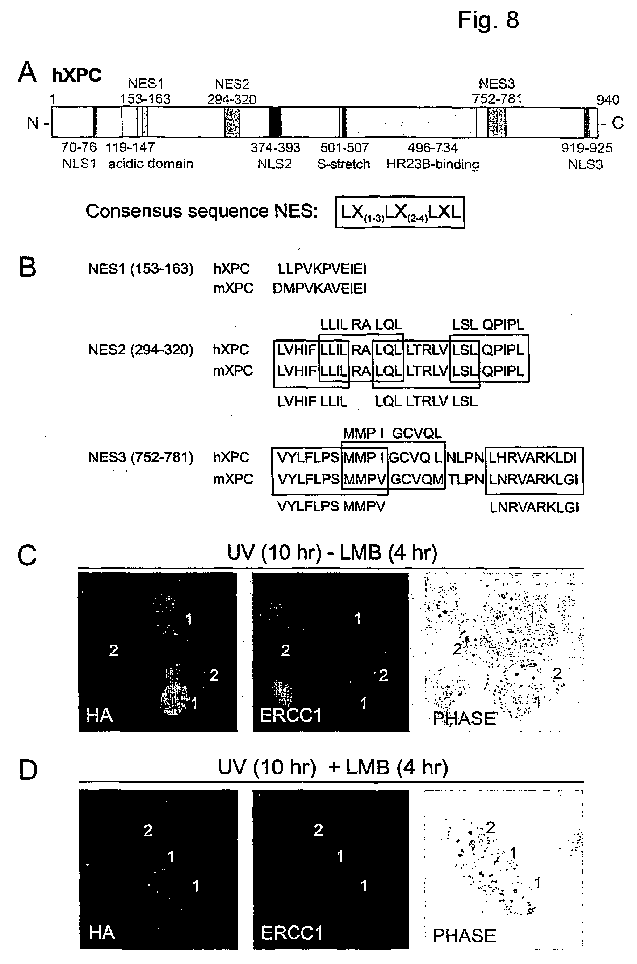

The findings above demonstrate that XPC levels are under tight control in an HR23-dependent fashion. Close inspection of the XPC sequence revealed several potential nuclear location (NLS) and nuclear export (NES) signals (provisionally referred to as NES1, NES2 and NES3, Figure 8A and B). We therefore investigated whether nuclear-cytoplasmic shuttling regulates XPC levels as reported for several other short-lived proteins, such as p53 and clock proteins (Sionov et al., 2001; Yagita et al., 2002) and whether DNA damage influences this process.

Nuclear export occurs via the chromosome region maintenance 1 (CRM1)/Exportinl system (Mattaj and Englmeier, 1998; Nigg, 1997). To investigate whether XPC shuttles between nucleus and cytoplasm, we studied the effect of leptomycin B (LMB), an established specific inhibitor of CRM1/Exportin1-mediated nuclear export (Fornerod et al., 1997; Fukuda et al., 1997) on the location of XPC-GFP. Using a heterokaryon nuclear-cytoplasmic shuttling assay (Borer et al., 1989) with *DKO* cells stably expressing *XPC-GFP*/*hHR23B* fused to human cells (HeLa) transport of the fluorescent protein (both in the presence and absence of 10 ng/ml LMB) from mouse nuclei to human nuclei can be monitored. Four hours after fusion, cells were immunostained with anti-HA monoclonals to identity the fusion protein (XPC-GFP-His₆HA) and specific human ERCC1 antibodies (that do not cross react to rodent ERCC1) to recognize HeLa nuclei. As shown in Figure 8C, 4 hr after fusion in the absence of LMB the nuclear pool of XPC-GFP induced by 10 J/m² UV-C light (given 6 hr prior to cell fusion) in the *DKO* cells was exchanged with non-irradiated human nuclei. Administration of LMB directly after cell fusion prevented this exchange (Figure 8D), showing that export from the mouse nuclei was responsible for the accumulation of the XPC-GFP in the untreated HeLa nuclei. A similar effect on the XPC-GFP shuttling was observed when XPC-GFP accumulation was provoked by 10 µM CBZ-LLL treatment (not shown). Parallel to the documented cases of p53 and clock proteins these findings demonstrate that proteolysis of XPG involves a nuclear-cytoplasmic shuttling mechanism.

### Generation of clones provided with murine HR23

The mHR23A/mHR23B double mutant cells are transfected with complementing functional mHR23A or mHR23B cDNAs tagged with versions of the GFP fluorescent marker to permit *in vivo* dynamic studies. Additionally the mouse HR23 cDNA's are provided with other tags facilitating purification on the basis of affinity chromatography. Because murine genes are used in this experiment full functional complementation is obtained avoiding possible interspecies differences and consequent incomplete or aberrant correction of a primary defect. In some of the transfections other genes/cDNAs known to be binding partners of HR23A or HR23B, such as XPC, MAG, p53, centrin tagged with compatible fluorescent markers and affinity tags are included in the transfection. Clones selected for stable expression of the co-transfected dominant selectable marker are screened for functional complementation of a HR23 defect and for proper expression of the co-transfected other gene. Clones are used for identifying the network regulated by the HR23 pathway and application for read-out of genotoxicity and for general cellular stress.

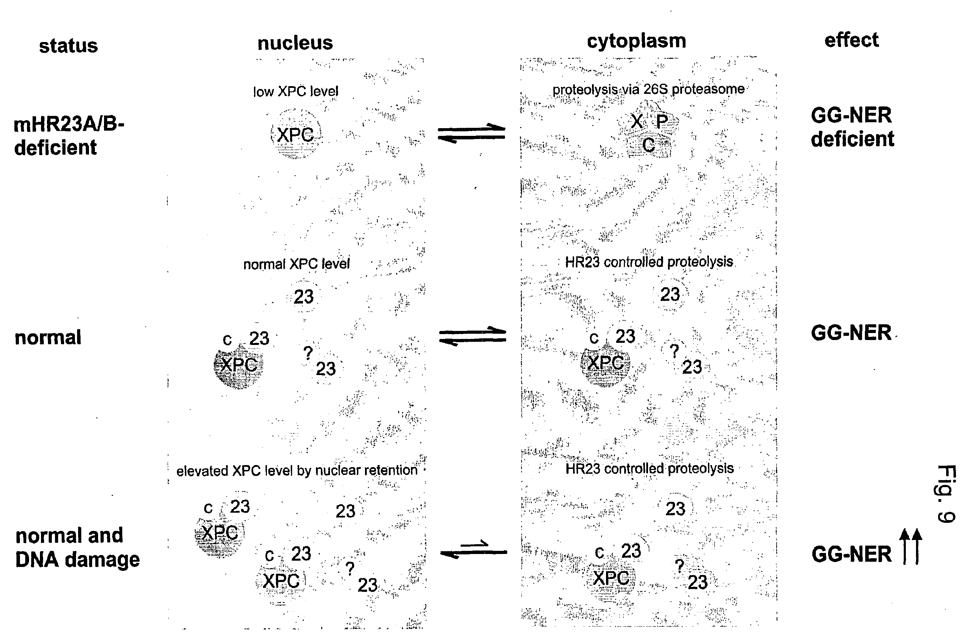

A unifying model for our findings on HR23, XPC and proteolysis is depicted in Figure 9. As the main initiator of GG-NER, XPC constitutes an ideal focal point for the regulation of the entire pathway, which involves HR23. Absence of HR23 proteins reveals that XPC on its own is highly unstable due to proteolysis via the 26S proteasome. Under normal conditions HR23 complex formation with XPC results in a significant reduction of XPC proteolysis and consequently in increased steady-state levels of the protein complex. This correlates with proficient GG-NER. Under conditions of a high level of DNA damage, involvement in NER stimulates the protective role of HR23. Particularly after prolonged higher damage load this leads to gradual up-regulation of XPC and consequently the entire GG-NER pathway. This rheostat model for adapting XPC levels to the amount of damage provides a novel type of regulation of DNA repair capacity in eukaryotes.

**Table 1. Genotype analysis of DKO (mHR23A^{-/-}/B^{-/}⁻) embryos and offspring**

| **Stage** | **Analyzed** | **Expected* (if Mendelian)** | **Found** |
|---|---|---|---|
| E8.5 | 43 | 7 | 3# |
| E10.5 | 14 | 1.8 | 0 |
| E13.5 | 77 | 9.1 | 0 |
| Newborn | 427 | 41.4 | 0 |

| | | | |
|---|---|---|---|
| * Derived from different *mHR23A*^{*+*/*-*} / *B*^{*+*/}*⁻* and *mHR23A*^{*-*/}*⁻lB*^{*+*/}*⁻* intercrosses # One cell line established | | | |

### Brief description of the drawings

Figure 1. Targeted disruption of the *mHR23A* gene by homologous recombination.
   (A) Genomic organization and disruption strategy for *mHR23A* depicting the gene, the targeting construct, and the targeted *mHR23A* allele. Exon III till VI (and part of exon II and VII) was replaced by the dominant selectable neomycin resistance marker transcribed in antisense orientation.
   (B) Southern blot analysis of BamHI digested DNA from ES cells showing the 5.0 kb and 3.5 kb fragment representing the wildtype and the targeted allele of *mHR23A,* respectively.
   (C) Southern blot analysis of BamHI digested tail DNA from *mBR23A*^{*+*/}*⁺, mHR23A*^{*+*/}*⁻,* and *mHR23A*^{*-*/}*⁻* mice.
   (D) RNA blot analysis of mHR23A mRNA from *mHR23A*^{*+*/}*⁺, mHR23A*^{*+*/}*⁻,* and *mHR23A*^{*-*/}*⁻* MEFs using *mHR23A* cDNA as a probe (upper panel). As a loading control for the amount of RNA, the blot was reprobed with β-actin cDNA (lower panel).
   (E) Immunoblot analysis of mHR23A protein in cellular extracts from *mHR23A*^{*+*/}*⁺, mHR23A*^{*+*/}*⁻,* and *mHR23A*^{*-*/}*⁻* MEFs loaded in equal amounts. Polyclonal antibodies against the human HR23A protein (upper panel) and the human XPC protein (lower panel) were utilized. The asterisk indicates an aspecific cross-reacting band.
Figure 2. Repair characteristics of *mHR23A*^{*-*/}*⁻* E13.5 and *DKO* E8.5 MEFs.
   (A) UV survival curves of primary *mHR23A*^{*+*/}*⁺, mHR23A*^{*+*/}*⁻,* and *mHR23A*^{*-*/*-*}E13.5 MEFs. *XPC*^{-/-} fibroblasts were included as negative control. Cells were exposed to different doses of UV (254 nm). After 4-5 days, the number of proliferating cells was estimated from the amount of radioactivity incorporated during a 3 hr pulse with [³H]thymidine. For each genotype, identical results were obtained with three other cell lines (data not shown).
   (B) Global genome repair (UDS) in primary *mHR23A*^{*+*/}*⁺, mHR23A*^{*+*/}*⁻,* and *mHR23A*^{*-*/}*⁻* E13.5 MEFs. Cells were irradiated with 16 J/m² UV (254 nm) and labeled with [³H]thymidine. Incorporation of radioactivity was measured by autoradiography and grain counting (average of 50 nuclei per cell line; the standard error of the mean is indicated). *XPA*^{-/-} fibroblasts were measured as negative control. For each genotype, consistent results were obtained with three other independent cell lines (data not shown).
   (C) RNA synthesis recovery (RRS) after UV-exposure of primary *mHR23A*^{*+*/}*⁺, mHR23A*^{*+*/}*⁻* and *mHR23A*^{*-*/}*⁻* E13.5 MEFs. Cells were UV-irradiated (10 J/m², 254 nm) and allowed to recover for 16 hours. After a 1 hr pulse labeling with [³H]uridine, cells were processed for autoradiography. The relative rate of RNA synthesis was expressed as the quotient of the number of autoradiographic grains over the UV-exposed nuclei and the number of grains over the nuclei of non-irradiated cells (average of 50 nuclei per cell line; the standard error of the mean is indicated). *CSB*^{*-*/}*⁻* cells were used as negative control. For each genotype, three other independent lines were assayed with similar outcome (data not shown).
   (D) UV survival of E8.5 MEF lines of wildtype, *XPC^{-l-}, mHR23A*^{*-*/*-*}/*B*^{*+*/}*⁻, mHR23A*^{*+*/*-*}/*B*^{*-*/}*⁻,* and *mHR23A*^{*-*/*-*}/*B*^{*-*/}*⁻* (*DKO*).
   (E) UV-induced UDS in wildtype, *XPC*^{-/-} and *DKO* E8.5 MEFs.
   (F) RNA synthesis recovery after UV-irradiation of wildtype, *XPC*^{-/-} and *DKO* E8.5 MEFs. For details for panels D-F see legends to panels A-C respectively and Experimental Procedures. Two independent experiments using two other *DKO* cell lines (before the cultures extinguished, not shown) showed a similar effect on UDS and RNA synthesis recovery.
Figure 3. XPC expression in *DKO* E8.5 MEFs.
   (A) Phase contrast (left panels) and epifluorescense (middle and right panels) images of fixed wildtype (WT, labeled with latex beads), *XPC*^{*-*/}*⁻* (*XPC*) and *mHR23A*^{*-*/*-*}/*B*^{*-*/}*⁻* (*DKO*) MEFs. Cells were fixed by paraformaldehyde, permeabilized by 0.1% triton X-100, and subsequently immunolabeled with affinity-purified polyclonal antibodies against the human XPC protein (middle panels; stained green with goat anti-rabbit Alexa 488-labeled secondary antibody). Monoclonal antibodies recognizing p62 subunit of TFIIH (right panels; stained red with goat anti-mouse Cy3-labeled secondary antibody) were used as an internal control. All images were taken at the same magnification.
   (B-C) Immunoblot analysis of XPC protein in cellular extracts from wildtype, *XPC*^{-/-} and *DKO* E8.5 MEFs using polyclonal anti-human XPC antibodies (B). Monoclonal anti-p62 antibodies were used as an internal reference for the amount of protein in each lane (C).
Figure 4. Characterization *of DKO* cells expressing hHR23B and XPC-GFP.
   (A) UV survival of wildtype, *XPC*^{*-*/}*⁻, DKO,* and *DKO* MEFs cotransfected with: *hHR23B (h23B),* human *XPC-GFP* (*hXPC*)*,* and *h23B* and *hXPC-GFP* cDNAs. Cells were exposed to different doses of UV (254 nm). After 4-5 days, the number of proliferating cells was estimated from the amount of radioactivity incorporated during a 3 hr pulse with [³H]thymidine. For details see Experimental Procedures. For each cDNA construct, similar results were obtained with at least two other independent stably transfected cell lines (data not shown).
   (B) Schematic representation of XPC-EGFP-His₆HA-N₃ fusion protein (1208 aa). Indicated are the human XPC protein (940 aa), the enhanced green fluorescent protein tag (EGFP; 238 aa), and the hexameric histidine-hemagglutinin double epitope tag (His₆HA; 17 aa).
   (C) Immunoblot analysis of XPC expression in cellular extracts of WT (lane 1), *XPC* (lane 2), *DKO* (lane 3), and *DKO* MEFs cotransfected with: *h23B* (lane 4), *hXPC-GFP* (lane 5), and *h23B* and *hXPC-GFP* (lane 6) cDNAs, using a polyclonal antibody against the C-terminus of human XPC (upper panel). Monoclonal anti-p62 antibodies were used as a loading control (lower panel).
   (D) Phase contrast (left) and epifluorescense (right) images of fixed WT (labeled with latex beads) and *DKO* cells cotransfected with *hHR23B* cDNA. Cells were fixed by paraformaldehyde, followed by 0.1% triton X-100 permeabilization and subsequently immunolabeled with affinity-purified polyclonal anti-human XPC (right; stained green with goat anti-rabbit Alexa 488-labeled secondary antibody). Monoclonal anti-p62 antiserum was used as an internal control (stained red with goat anti-mouse Cy3-labeled secondary antibody; data not shown). Images were taken at the same magnification. Similar results were obtained with *DKO* cells cotransfected with *hXPC-GFP,* and *hHR23B* and *hXPC-GFP* cDNAs (not shown).
   (E-F) Phase contrast (left panels) and epifluorescense (right panels) images of living *DKO* cells cotransfected with: *HXPC-GFP* (E), or *hHR23B* and *hXPC-GFP* (F) cDNAs. All images were taken at the same magnification.
Figure 5. Effect of UV, NA-AAF, and proteasome inhibitor on hHR23B-dependent XPC-GFP level in living *DKO* cells
   (A) Kinetic analysis of living *DKO* cells expressing XPC-GFP/hHR23B upon 10 J/m² UV-C in time over a period of 30 hours. Percentage XPC-GFP: the percentage of GFP-expressing fluorescent cells of the total number of cells.
   (B) Immunoblot analysis *of DKO* cells expressing XPC-GFP/hHR23B before exposure to damaging agent (lane 1), 6 hr after exposure to 10 J/m² UV-C (lane 2), and 6 hr after treatment with 10 µM CBZ-LLL (lane 3) using monoclonal antibodies recognizing the HA epitope of XPC-GFP (upper panel). A monoclonal antibody against the p62 subunit of TFIIH (lower panel) was used as a loading control. A similar outcome was obtained with two other independent *DKO* cell lines expressing XPC-GFP/hHR23B (data not shown).
   (C) Combined phase contrast (red) and fluorescence (green) images (upper panels), and epifluorescence images (lower panels) of the same living *DKO* cells expressing XPC-GFP/hHR23B before UV (left panels) and 6 hr after 10 J/m² UV-C (right panels). White arrows indicate the scratch mark on glass coverslips. Numbers represent the same living cells before and after UV-exposure. Identical results were obtained with two other independent *DKO* cell lines expressing XPC-GFP/hHR23B (data not shown). All images were taken at the same magnification.
   (D) Combined phase contrast (red) and fluorescence (green) images (upper panels), and epifluorescence images (lower panels) of living *DKO* cells expressing XPC-GFP/hHR23B before NA-AAF (left panels) and 8 hr after 50 µM NA-AAF (right panels). White arrows indicate the scratch on glass coverslips. The numbers represent the corresponding living cells on coverslips before and after NA-AAF treatment. Identical results were obtained with two other independent *DKO* cell lines expressing XPC-GFP/hHR23B (data not shown). All images were taken at the same magnification.
   (E) Combined phase contrast (red) and fluorescence (green) images (upper panels), and only epifluorescence images (lower panels) of living *DKO* cells expressing XTC-GFP/hHR23B before treatment with proteasome inhibitor CBZ-LLL (left panels) and 6 hr after 10 µM CBZ-LLL (right panels). All images were taken at the same magnification.
Figure 6. Local UV damage induces overall XPC stabilization in nuclei of *DKO* cells expressing XPC-GFP/hHR23B.
   (A-B) *DKO* cells expressing XPC-GFP/hHR23B were exposed to 64 J/m² UV-C through 5.0 µm pore filters and fixed 5 min (A) and 2 hours (B) later with paraformaldehyde. Double immunofluorescent labeling using antibodies against XPA (A, left panel; stained green with goat anti-rabbit Alexa 488-labeled secondary antibody) and HA epitope (A, right panel; stained red with goat anti-rat Alexa 594-labeled secondary antibody). DAPI stained (B, left panel) and epifluorescence images without antibody labeling (B, right panel). Arrows indicate the site of UV-induced local damage in the nuclei of *DKO* cells expressing hXPC-GFP/hHR23B. Note: Compare the increased fluorescence signal over the entire nucleus of damaged cells to the signal of non-damaged nuclei for overall stabilization of XPC (B).
Figure 7. Enhanced DNA repair correlates with high levels of XPC in UV-induced UDS in *DKO* cells expressing XPC-GFP/hHR23B.
   (A) Histogram of UV-induced UDS in *DKO* cells expressing XPC-GFP/hHR23B. 5 hr after exposure to 10 J/m² UV-C, cells were subsequently irradiated with 16 J/m² UV-C and labeled with [³H]thymidine for 1 hr (white columns, mean of UDS level is 25 ± SEM 1). In parallel, non-prechallenged cells only exposed to 16 J/m² UV-C were used as controls (black columns, mean of LTDS level is 16 ± SEM 0.6). Asterisks indicate the mean values of the UDS levels. Incorporation of radioactivity was measured by autoradiography and grain counting (130 fixed squares counted per cell line and each square represented approximately 50% of the nucleus surface). UV-induced UDS of wildtype (mean 17 ± SEM 0.8) fibroblasts were measured as controls (data not shown).
   (B) Effect of microinjection of *XPC-GFP* cDNA on UV-induced UDS in human wildtype (C5RO) fibroblasts. Shown is a micrograph of a wildtype homodikaryon (numbered 1) microinjected with *XPC-GFP* in one of the nuclei and subjected to UV-induced UDS. Prior to UDS, fluorescence images were captured (inset in B). The injected cell has a considerably larger number of grains above its nuclei than the noninjected, surrounding mononuclear cells (numbered 2).
Figure 8. Evidence for XPC shuttling between nucleus and cytoplasm.
   (A) Schematic representation of human XPC protein (940 aa), indicating three putative leucine-rich nuclear export signal (NES) in red, three putative nuclear location signals (NLS) in blue, an N-terminal acidic stretch, a central Serine rich domain, and a C-terminal HR23-binding region (Uchida et al., 2002). The consensus sequence for NES is indicated separately. Although originally defined as leucine-rich, other hydrophobic residues (I, F, V, M) have been shown to be able to substitute for leucines in functional NES sequences of various proteins (Mowen and David, 2000; Roth et al., 1998).
   (B) Amino acid sequence comparison between mouse and human XPC NES-like domains. Numbers indicate the location of the amino acids within the respective proteins. Closer examination of NES2 and NES3 revealed multiple conserved leucine-rich regions.
   (C-D) Heterokaryon nuclear-cytoplasmic shuttling assay using *DKO* cells expressing XPC-GFP(His₆HA)/hHR23B and HeLa cells. Six hours prior to cell fusion, cells were exposed to 10 J/m² UV-C. After cell fusion, cells were cultured either in the absence (C) or in the presence of the nuclear export inhibitor LMB (10 ng/ml) (D). Four hours after fusion cells were fixed, and immunostained with anti-HA (left) to detect the fusion protein and anti-hERCCl-specific antibody (middle) to recognize HeLa cells. The right picture is a phase contrast image of the same cells. For clarity, mouse nuclei were marked by (1) and human (HeLa) nuclei were marked by (2).
Figure 9. Model for the DNA damage and HR23-dependent regulation of XPC and GG-NER.
   In the total absence of the HR23 proteins (*mHR23A*/*B*-decifient), XPC is intrinsically unstable and targeted for ubiquitin-dependent proteolysis via the 26S proteasome. In view of the parallel with p53 nucleo-cytoplasmic shuttling we postulate that XPC is degraded in the cytoplasm. As a consequence the steady-state level of XPC is decreased resulting in reduced GG-NER capacity (upper panel). Under normal conditions, HR23 proteins (indicated as 23) control XPC degradation leading to partial stabilization of XPC (in a complex with HR23 and CEN2 (C)). Higher steady-state levels of XPC result in proficient GG-NER (middle panel). NER-type DNA damage (e.g. UV-irradiation) induces a further increase in XPC/HR23/CEN2 protein levels through nuclear retention of XPC bound to lesions, and accordingly enhances GG-NER capacity (lower panel). A comparable HR23-mediated stabilization mechanism may hold for other factors (here indicated by "?") and cellular pathways in which HR23 proteins are implicated (see discussion for further explanation).

### References

Araki, M., Masutani, C., Takemura, M., Uchida, A., Sugasawa, K., Kondoh, J., Ohkuma, Y., and Hanaoka, F. (2001). Centrosome protein centrin 2/caltractin 1 is part of the xeroderma pigmentosum group C complex that initiates global genome nucleotide excision repair. J Biol Chem 18665-18672, 27.
Bertolaet, B. L., Clarke, D. J., Wolff, M., Watson, M. H., Henze, M., Divita, G., and Reed, S. 1. (2001). UBA domains of DNA damage-inducible proteins interact with ubiquitin. Nat Struct Biol 8, 417-422.
Biggins, S., Ivanovska, I., and Rose, M. D. (1996). Yeast ubiquitin-like genes are involved in duplication of the microtubule organizing center. J Cell Biol 133; 1331-1346.
Bootsma, D., Kraemer, K. H., Cleaver, J. E., and Hoeijmakers, J. H. J. (2001). Nucleotide excision repair syndromes: xeroderma pigmentosum, Cockayne syndrome, and trichothiodystrophy. In Scriver CR, Beaudet, AL, Sly, WS, Valle, D Vogelstein and Kinzler (eds). The Metabolic and Molecular Bases of Inherited Disease McGraw-Hill Book Co, New York 1, 677-703.
Borer, R. A., Lehner, C. F., Eppenberger, H. M., and Nigg, E. A. (1989). Major nucleolar proteins shuttle between nucleus and cytoplasm. Cell 56, 379-390.
Chen, L., and Madura, K. (2002). Rad23 promotes the targeting of proteolytic substrates to the proteasome. Mol Cell Biol 22, 4902-4913.
Chen, L., Shinde, U., Ortolan, T. G., and Madura, K. (2001). Ubiquitin-associated (UBA) domains in Rad23 bind ubiquitin and promote inhibition of multi-ubiquitin chain assembly. EMBO Rep 2, 933-938.
Clarke, D. J., Mondesert, G., Segal, M., Bertolaet, B. L., Jensen, S., Wolff, M., Henze, M., and Reed, S. I. (2001). Dosage suppressors of pds1 implicate ubiquitin-associated domains in checkpoint control. Mol Cell Biol 21, 1997-2007.
Fornerod, M., Ohno, M., Yoshida, M., and Mattaj, I. W. (1997). CRM1 is an export receptor for leucine-rich nuclear export signals. Cell 90, 1051-1060.
Friedberg, E. C., Walker, G. C., and Siede, W. (1995). DNA repair and mutagenesis. ASM Press, Washington DC.
Fukuda, M., Asano, S., Nakamura, T., Adachi, M., Yoshida, M., Yanagida, M., and Nishida, E. (1997). CRM1 is responsible for intracellular transport mediated by the nuclear export signal. Nature 390, 308-311.
Gillette, T. G., Huang, W., Russell, S. J., Reed, S. H., Johnston, S. A., and Friedberg, E. C. (2001). The 19S complex of the proteasome regulates nucleotide excision repair in yeast. Genes Dev 15, 1528-1539.
Gurtner, G. C., Davis, V., Li, H., McCoy, M. J., Sharpe, A., and Cybulsky, M. I. (1995). Targeted disruption of the murine VCAM1 gene: essential role of VCAM-1 in chorioallantoic fusion and placentation. Genes Dev 9, 1-14.
Hanawalt, P. C. (2000). DNA repair. The bases for Cockayne syndrome.
Hardeland, U., Steinacher, R., Jiricny, J., and Schar, P. (2002). Modification of the human thymine-DNA glycosylase by ubiquitin-like proteins facilitates enzymatic turnover. Embo J 21, 1456-1464.
Hiyama, H., Yokoi, M., Masutani, C., Sugasawa, K., Maekawa, T., Tanaka, K., Hoeijmakers, J. H. J., and Hanaoka, F. (1999). Interaction of hHR23 with s5a. The ubiquitin-like domain of hhr23 mediates interaction with s5a subunit of 26s proteasome. J Biol Chem 274, 28019-28025.
Hoeijmakers, J. H. J. (2001). Genome maintenance mechanisms for preventing cancer. Nature 411, 366-374.
Houtsmuller, A. B., Rademakers, S., Nigg, A. L., Hoogstraten, D., Hoeijmakers, J. H. J., and Vermeulen, W. (1999). Action of DNA repair endonuclease ERCC1/XPF in living cells. Science 284, 958-961.
Kumar, S., Talis, A. L., and Howley, P. M. (1999). Identification of HHR23A as a substrate for E6-associated protein-mediated ubiquitination. J Biol Chem 274, 18785-18792.
Lambertson, D., Chen, L., and Madura, K. (1999). Pleiotropic defects caused by loss of the proteasome-interacting factors Rad23 and Rpn10 of Saccharomyces cerevisiae. Genetics 153, 69-79.
Lombaerts, M., Goeloe, J. I., den Dulk, H., Brandsma, J. A., and Brouwer, J. (2000). Identification and characterization of the rhp23(+) DNA repair gene in Schizosaccharomyces pombe. Biochem Biophys Res Commun 268, 210-215.
Lommel, L., Chen, L., Madura, K., and Sweder, K. (2000). The 26S proteasome negatively regulates the level of overall genomic nucleotide excision repair. Nucleic Acids Res 28, 4839-4845.
Masutani, C., Araki, M., Sugasawa, K., van der Spek, P. J., Yamada, A., Uchida, A., Maekawa, T., Bootsma, D., Hoeijmakers, J. H. J., and Hanaoka, F. (1997). Identification and characterization of XPC-binding domain of hHR23B. Mol Cell Biol 17, 6915-6923.
Masutani, C., Sugasawa, K., Yanagisawa, J., Sonoyama, T., Ui, M., Enomoto, T., Takio, K., Tanaka, K., van der Spek, P. J., Bootsma, D., and et al. (1994). Purification and cloning of a nucleotide excision repair complex involving the xeroderma pigmentosum group C protein and a human homologue of yeast RAD23. Embo J 13, 1831-1843.
Mattaj, I. W., and Englmeier, L. (1998). Nucleocytoplasmic transport: the soluble phase. Annu Rev Biochem 67, 265-306.
Memisoglu, A., and Samson, L. (2000). Base excision repair in yeast and mammals. Mutat Res 451, 39-51.
Miao, F., Bouziane, M., Dammann, R., Masutani, C., Hanaoka, F., Pfeifer, G. P., and O'Connor, T. R. (2000). 3-methyladenine-DNA glycosylase (MPG protein) interacts with human RAD23 proteins. J Biol Chem 275, 28433-28438.
Mowen, K., and David, M. (2000). Regulation of STAT1 nuclear export by Jakl. Mol Cell Biol 20, 7273-7281.
Ng, J. M. Y., Vrieling, H., Sugasawa, K., Ooms, M. P., Grootegoed, J. A., Vreeburg, J. T., Visser, P., Beems, R. B., Gorgels, T. G., Hanaoka, F., et al. (2002). Developmental defects and male sterility in mice lacking the ubiquitin-like DNA repair gene mHR23B. Mol Cell Biol 22, 1233-1245.
Nigg, E. A. (1997). Nucleocytoplasmic transport: signals, mechanisms and regulation. Nature 386, 779-787.
Ortolan, T. G., Tongaonkar, P., Lambertson; D., Chen, L., Schauber, C., and Madura, K. (2000). The DNA repair protein Rad23 is a negative regulator of multi-ubiquitin chain assembly. Nat Cell Biol 2, 601-608.
Rao, H., and Sastry, A. (2002). Recognition of specific ubiquitin conjugates is important for the proteolytic functions of the UBA domain proteins Dsk2 and Rad23. J Biol Chem 22.
Roth, J., Dobbelstein, M., Freedman, D. A., Shenk, T., and Levine, A. J. (1998). Nucleo-cytoplasmic shuttling of the hdm2 oncoprotein regulates the levels of the p53 protein via a pathway used by the human immunodeficiency virus rev protein. Embo J 17, 554-564.
Russell, S. J., Reed, S. H., Huang, W., Friedberg, E. C., and Johnston, S. A. (1999). The 19S regulatory complex of the proteasome functions independently of proteolysis in nucleotide excision repair. Mol Cell 3, 687-695.
Schauber, C., Chen, L., Tongaonkar, P., Vega, I., Lambertson, D., Potts, W., and Madura, K. (1998). Rad23 links DNA repair to the ubiquitin/proteasome pathway. Nature 391, 715-718.
Siede, W., and Eckardt-Schupp, F. (1986). DNA repair genes of Saccharomyces cerevisiae: complementing rad4 and reu2 mutations by plasmids which cannot be propagated in Escherichia coli. Curr Genet 11, 205-210.
Sionov, R. V., Coen, S., Goldberg, Z., Berger, M., Bercovich, B., Ben-Neriah, Y., Ciechanover, A., and Haupt, Y. (2001). c-Abl regulates p53 levels under normal and stress conditions by preventing its nuclear export and ubiquitination. Mol Cell Biol 21, 5869-5878.
Sugasawa, K., Okamoto, T., Shimizu, Y., Masutani, C., Iwai, S., and Hanaoka, F. (2001). A multistep damage recognition mechanism for global genomic nucleotide excision repair. Genes Dev 15, 507-521.
Sugasawa, K., Ng, J. M. Y., Masutani, C., Iwai, S., van der Spek, P. J., Eker, A. P., Hanaoka, F., Bootsma, D., and Hoeijmakers, J. H. J. (1998). Xeroderma pigmentosum group C protein complex is the initiator of global genome nucleotide excision repair. Mol Cell 2, 223-232.
Sugasawa, K., Ng, J. M. Y., Masutani, C., Maekawa, T., Uchida, A., van der Spek, P. J., Eker, A. P., Rademakers, S., Visser, C., Aboussekhra, A., et al. (1997). Two human homologs of Rad23 are functionally interchangeable in complex formation and stimulation of XPC repair activity. Mol Cell Biol 17, 6924-6931.
Sugasawa, K., Masutani, C., Uchida, A., Maekawa, T., van der Spek, P. J., Bootsma, D., Hoeijmakers, J. H. J., and Hanaoka, F. (1996). HHR23B, a human Rad23 homolog, stimulates XPC protein in nucleotide excision repair in vitro. Mol Cell Biol 16, 4852-4861.
Sutton, M. D., Smith, B. T., Godoy, V. G., and Walker, G. C. (2000). The SOS response: recent insights into umuDC-dependent mutagenesis and DNA damage tolerance. Annu Rev Genet 34, 479-497.
Uchida, A., Sugasawa, K., Masutani, C., Dohmae, N., Araki, M., Yokoi, M., Ohkuma, Y., and Hanaoka, F. (2002). The carboxy-terminal domain of the XPC protein plays a crucial role in nucleotide excision repair through interactions with transcription factor IIH. DNA Repair 1, 449-461.
van der Spek, P. J., Eker, A., Rademakers, S., Visser, C., Sugasawa, K., Masutani, C., Hanaoka, F., Bootsma, D., and Hoeijmakers, J. H. J. (1996a). XPC and human homologs of RAD23: intracellular localization and relationship to other nucleotide excision repair complexes. Nucleic Acids Res 24, 2551-2559.
van der Spek, P. J., Visser, C. E., Hanaoka, F., Smit, B., Hagemeijer, A., Bootsma, D., and Hoeijmakers, J. H. J. (1996b). Cloning, comparative mapping, and RNA expression of the mouse homologues of the Saccharomyces cerevisiae nucleotide excision repair gene RAD23. Genomics 31, 20-27.
Venema, J., van Hoffen, A., Natarajan, A. T., van Zeeland, A. A., and Mullenders, L. H. (1990). The residual repair capacity of xeroderma pigmentosum complementation group C fibroblasts is highly specific for transcriptionally active DNA. Nucleic Acids Res 18, 443-448.
Verhage, R. A., Zeeman, A. M., Lombaerts, M., van de Putte, P., and Brouwer, J. (1996). Analysis of gene- and strand-specific repair in the moderately UV-sensitive Saccharomyces cerevisiae rad23 mutant. Mutat Res 362, 155-165*.*
Vermeulen, W., Scott, R. J., Rodgers, S., Muller, H. J., Cole, J., Arlett, C. F., Kleijer, W. J., Bootsma, D., Hoeijmakers, J. H. J., and Weeda, G. (1994). Clinical heterogeneity within xeroderma pigmentosum associated with mutations in the DNA repair and transcription gene ERCC3. Am J Hum Genet 54, 191-200.
Volker, M., Moné, M. J., Karmakar, P., Hoffen, A., Schul, W., Vermeulen, W., Hoeijmakers, J. H. J., van Driel, R., Zeeland, A. A., and Mullenders, L. H. F. (2001). Sequential assembly of the nucleotide excision repair factors in vivo. Mol Cell 8, 213-224.
Watkins, J. F., Sung, P., Prakash, L., and Prakash, S. (1993). The Saccharomyces cerevisiae DNA repair gene RAD23 encodes a nuclear protein containing a ubiquitin-like domain required for biological function. Mol Cell Biol 13, 7757-7765.
Wei, S., and Friedberg, E. C. (1998). A fragment of the yeast DNA repair protein Rad4 confers toxicity to E. coli and is required for its interaction with Rad7 protein. Mutat Res 400, 127-133.
Wiertz, E. J., Jones, T. R., Sun, L., Bogyo, M., Geuze, H. J., and Ploegh, H. L. (1996). The human cytomegalovirus US11 gene product dislocates MHC class I heavy chains from the endoplasmic reticulum to the cytosol. Cell 84, 769-779.
Wilkinson, C. R., Seeger, M., Hartmann-Petersen, R., Stone, M., Wallace, M., Semple, C., and Gordon, C. (2001). Proteins containing the UBA domain are able to bind to multi-ubiquitin chains. Nat Cell Biol 3, 939-943.
Wood, R. D., Hanawalt, P. C., Mer, G., and Cooper, P. K. (2001a). Nucleotide excision repair. In Biological responses to DNA damage. Cold Spring Harb Symp Quant Biol LXV.
Wood, R. D., Mitchell, M., Sgouros, J., and Lindahl, T. (2001b). Human DNA repair genes. Science 291, 1284-1289.
Yagita, K., Tamanini, F., Yasuda, M., Hoeijmakers, J. H., van der Horst, G. T., and Okamura, H. (2002). Nucleocytoplasmic shuttling and mCRY-dependent inhibition of ubiquitylation of the mPER2 clock protein. Embo J 21, 1301-1314.
Yokoi, M., Masutani, C., Maekawa, T., Sugasawa, K., Ohkuma, Y., and Hanaoka, F. (2000). The xeroderma pigmentosum group C protein complex XPC-HR23B plays an important role in the recruitment of transcription factor IIH to damaged DNA. J Biol Chem 275, 9870-9875.
Zhu, Q., Wani, G., Wani, M. A., and Wani, A. A. (2001). Human homologue of yeast Rad23 protein A interacts with p300/cyclic AMP-responsive element binding (CREB)-binding protein to down-regulate transcriptional activity of p53. Cancer Res 61, 64-70.

## Claims

1. An vitro method for determining whether an agent is capable of inducing a DNA lesion that is substrate for global genome nucleotide excision repair in a eukaryotic cell, the method comprising:
- exposing at least one eukaryotic cell to said agent, and
- determining whether an overall intranuclear accumulation of xeroderma pigmentosum group C protein, or a functional part, thereof which has the same properties as XPC, occurs within said cell.

2. A method according to claim 1, wherein said cell is overexpressing HR23A and/or HR23B protein.

3. A method according to anyone of claims 1-2, wherein said cell is a mammalian cell.

4. A method according to anyone of claims 1,-3, wherein said cell is a murine cell.

5. A method according to anyone of claims 1-4, wherein said cell is a mouse embryonic fibroblast.

6. A method according to anyone of claims 1-5 wherein said cell is deficient in endogenous HR23A protein.

7. A method according to anyone of claims 1-6 wherein said cell is deficient in endogenous HR23B protein.

8. A method according to anyone of claims 1-7, wherein said cell is part of a cell line.

9. A method according to claim 8, wherein said cell line comprises a mouse embryonic fibroblast cell line.

10. A method according to claim 8 or 9, wherein the cells of said cell line are deficient in endogenous HR23A protein.

11. A method according to anyone of claims 8-10, wherein the cells of said cell line are deficient in endogenous HR23B protein.

12. A method according to anyone of claims 1-11, wherein said cell is provided with a nucleic acid encoding human HR23A protein.

13. A method according to anyone of claims 1-12, wherein said cell is provided with a nucleic acid encoding murine HR23A protein.

14. A method according to anyone of claims 1-13, wherein said cell has been provided with a nucleic acid encoding human HR23B protein.

15. A method according to anyone of claims 1-14, wherein said cell has been provided with a nucleic acid encoding murine HR23B protein.

16. A method According to anyone of claims 1-16, wherein said xeroderma pigmentosum group C protein comprises a human xeroderma pigmentosum group C protein.

17. A method according to anyone of claims 1-16, wherein said xeroderma pigmentosum group C protein or functional part, derivative and/or analogue thereof comprises a label.

18. A method according to claim 17, wherein said label comprises green fluorescent protein or luciferase.

19. A method for determining whether an agent is capable of at least in part inhibiting a cellular process, said process resulting in overall intranuclear accumulation of xeroderma pigmentosum group C protein, or a functional part, thereof which has the same properties as XPC, within a cell, the method comprising:
- exposing at least one eukaryotic cell to said agent, and
- determining whether an overall intranuclear accumulation of xeroderma pigmentosum group C protein, or a functional part, derivative and/or analogue thereof which has the same kind of properties as XPC, occurs within said cell.

20. A method according to claim 19, wherein said eukaryotic cell comprises a DNA lesion.

21. A method according to claim 19 or 20, wherein said process comprises proteolysis, nucleo-cytoplasma shuttling, RNA synthesis, RNA processing, RNA transport, and/or RNA translation.

22. A method according to claim 21, wherein said proteolysis comprises proteasomal proteolysis.

23. A method for determining whether a cell has an at least partly impaired global genome nucleotide excision repair system, comprising:
- exposing said cell to an agent capable of inducing a DNA lesion, and
- determining whether an overall intranuclear accumulation of xeroderma pigmentosum group C protein, or a functional part, thereof which has the same properties as XPC, occurs within said cell.

24. A method for determining whether an individual suffers from, or is at risk of suffering from, a disease related to an at least partly impaired global genome nucleotide excision repair system, comprising:
- exposing at least one cell having been obtained from said individual to an agent capable of inducing a DNA lesion, and
- determining whether an overall intranuclear accumulation of xeroderma pigmentosum group C protein, or a functional part, thereof which has the same properties as XPC, occurs in the nucleus of said cell.

25. A method according to claim 23 or 24, wherein said disease comprises xeroderma pigmentosum, Cockayne syndrome, and/or trichothiodystrophy.

26. A kit of parts comprising a eukaryotic cell which is deficient in endogenous HR23A protein and/or endogenous HR23B protein and an agent capable of inducing a DNA lesion that is substrate for global genome nucleotide excision repair and/or base excision repair.

27. A kit of parts according to claim 26, further comprising a detection system for detecting a change in level of xeroderma pigmentosum group C protein and/or 3-methyladenine DNA glycosylase, or a functional part, thereof.

## Patentansprüche

1. In vitro-Verfahren zur Bestimmung, ob ein Mittel in der Page ist, eine DNA-Läsion zu induzieren, die ein Substrat für eine globale Genom-Nukleotid-Exzisionreparatur in einer eukaryontischen Zelle ist, wobei das Verfahren Schritte umfasst, bei denen man:
- mindestens eine eukaryontische Zelle diesem Mittel aussetzt, und
- bestimmt, ob innerhalb der Zelle eine intranukleäre Gesamtakkumulation des Xeroderma pigmentosum-Gruppe C-Proteins oder eines funktionellen Teils davon, der die gleichen Eigenschaften wie XPC aufweiset, auftritt.

2. Verfahren nach Anspruch 1, bei dem die Zelle HR23A- und/oder HR23B-Protein überexprimiert.

3. Verfahren nach einem der Ansprüche 1 bis 2, bei dem die Zelle eine Säugetier-Zelle ist.

4. Verfahren nach einem der Ansprüche 1 bis 3, bei dem die Zelle eine murine Zelle ist.

5. Verfahren nach einem der Ansprüche 1 bis 4, bei dem die Zelle ein embryonischer Fibroblast der Maus ist.

6. Verfahren nach einem der Ansprüche 1 bis 5, bei dem die Zelle in Bezug auf endogenes HR23A-Protein defizient ist.

7. Verfahren nach einem der Ansprüche 1 bis 6, bei dem die Zelle in Bezug auf endogenes HR23B-Protein defizient ist.

8. Vorfahren nach einem der Ansprüche 1 bis 7, bei dem die Zelle Teil einer Zelllinie ist.

9. Verfahren nach Anspruch 8, bei dem die Zelllinie eine embryonische Fibroblasten-Zelllinie der Maus umfasst.

10. Verfahren nach Anspruch 8 oder 9, bei dem die Zellen der Zelllinie in Bezug auf endogenes HR23A-Protein defizient sind.

11. Verfahren nach einem der Ansprüche 8 bis 10, bei dem die Zellen der Zelllinie in Bezug auf endogenes HR23B-Protein defizient sind.

12. Verfahren nach einem der Ansprüche 1 bis 11, bei dem die Zelle mit einer Nukleinsäure ausgestattet wird, die für humanes HR23A-Protein kodiert.

13. Verfahren nach einem der Ansprüche 1 bis 12, bei dem die Zelle mit einer Nukleinsäure ausgestattet wird, die für murines HR23A-Protein kodiert.

14. Verfahren nach einem der Ansprüche 1 bis 13, bei dem die Zelle mit einer Nukleinsäure ausgestattet worden ist, die für humanes HR23B-Protein kodiert.

15. Verfahren nach einem der Ansprüche 1 bis 14, bei dem die Zelle mit einer Nukleinsäure ausgestattet worden ist, die für murines HR23B-Protein kopiert.

16. Verfahren nach einem der Ansprüche 1 bis 15,- bei dem das Xeroderma pigmentosum-Gruppe C-Protein ein humanes Xeroderma pigmentosum-Gruppe C-Protein umfasst.

17. Verfahren nach einem der Ansprüche 1 bis 16, bei dem das Xeroderma pigmentosum-Gruppe C-Protein oder ein funktionenler Teil, ein Derivat und/oder ein Analog davon eine Markierung umfasst.

18. Verfahren nach Anspruch 17, bei dem die Markierung grün fluoreszierendes Protein oder Luciferase umfasst.

19. Verfahren zur Bestimmung, ob ein Mittel in der Lage ist, zumindest teilweise einen zellulären Prozess zu inhibieren, wobei der Prozess innerhalb der Zelle zu einer intranukleären Gesamtakkumulation von Xeroderma pigmentosum-Gruppe C-Protein oder eines funktionellen Teils davon, der dieselben Eigenschaften wie XPC aufweist, führt, bei dem main:
- mindestens eine eukaryontische Zelle diesem Mittel aussetzt, und
- bestimmt, ob innerhalb dieser Zelle eine intranukleäre Gesamtakkumulation von Xeroderma pigmentosum-Gruppe C-Protein oder eines funktionellen Teils, eines Derivats und/oder eines Analogs davon, der/das dieselbe Art von Eigenschaften wie XPC aufweist, auftritt.

20. Verfahren nach Anspruch 19, bei dem die eukaryontische Zelle eine DNA-Läsion umfasst.

21. Verfahren nach Anspruch 19 oder 20, bei dem der Prozess eine Proteolyse, ein nukleozytoplasmatisches Shuttling, RNA-Synthese, RNA-Prozessierung, RNA-Transport und/oder RNA-Translation umfasst.

22. Verfahren nach Anspruch 21, bei dem die Proteolyse eine proteasomale Proteolyse umfasst.

23. Verfahren zur Bestimmung, ob eine Zelle ein zumindest teilweise beeinträchtigtes globales genomisches Nukleotid-Exzisionsreparatursystem aufweist, bei dem man:
- die Zelle einem Mittel aussetzt, das in der Lage ist, eine DNA-Läsion zu induzieren, und
- bestimmt, ob innerhalb der Zelle eine intranukleäre Gesamtakkumulation von Xeroderma pigmentosum-Gruppe C-Protein oder eines funktionellen Teils davon, der dieselben Eigenschaften wie XPC aufweist, auftritt.

24. Verfahren zur Bestimmung, ob ein Individium an einer Krankheit leidet oder ein Risiko dafür aufweist, an einer Krankheit zu leiden, wobei die Krankheit zumindest teilweise auf ein beeinträchtigtes globales genomisches Nukleotid-Exzisionsreparatursystem zurückzuführen ist, bei dem man:
- mindestens eine Zelle, die von dem Individuum erhalten wurde, einem Mittel aussetzt, das in der Lage ist, eine DNA-Läsion zu induzieren, und
- bestimmt, ob im Nukleus der Zelle eine intranukleäre Gesamtakkumulation von Xeroderma pigmentosum-Gruppe C-Protein oder eines funktionellen Teils davon, der dieselben Eigenschaften wie XPC aufweist, auftritt.

25. Verfahren nach Anspruch 23 oder 24, wobei die Erkrankung Xeroderma pigmentosum, Cockayne-Syndrom und/oder Trichothiodystrophie umfasst.

26. Einzelteile umfassendes Kit (kit of parts), das eine eukaryontische Zelle umfasst, welche in Bezug auf endogenes HR23A-Protein und/oder endogenes HR23B-Protein defizient ist, sowie ein Mittel, das in der Lage ist, eine DNA-Läsion zu induzieren, welche ein Substrat für ein globales genomisches Nukleotid-Exzisionsreparatur und/oder eine Blasen-Exzisionsreparatur ist.

27. Einzelteile umfassendes Kit (kit of parts) nach Anspruch 26, der ferner ein Nachweissystem für den Nachweis einer Veränderung der Menge von Xeroderma pigmentosum-Gruppe C-Protein und/oder 3-Methyladenin-DNA-Glykosylase oder eines funktionellen Teils davon umfasst.

## Revendications

1. Procédé *in vitro* pour déterminer si un agent est capable d'induire une lésion de l'ADN qui est un substrat pour une réparation globale du génome par excision de nucléotides dans une cellule eucaryote, le procédé comprenant les étapes consistant à :
- exposer au moins une cellule eucaryote audit agent, et
- déterminer si une accumulation intranucléaire globale de protéine du groupe C de *xeroderma pigmentosum,* ou d'une partie fonctionnelle de celle-ci qui présente les mêmes propriétés que la protéine XPC, se produit dans ladite cellule.

2. Procédé selon la revendication 1, dans lequel ladite cellule surexprime les protéines HR23A et/ou HR23B.

3. Procédé selon l'une quelconque des revendications 1 à 2, dans lequel ladite cellule est une cellule de mammifère.

4. Procédé selon l'une quelconque des revendications 1 à 3, dans lequel ladite cellule est une cellule murine.

5. Procédé selon l'une quelconque des revendications 1 à 4, dans lequel ladite cellule est un fibroblaste embryonnaire de souris.

6. Procédé selon l'une quelconque des revendications 1 à 5, dans lequel ladite cellule est déficiente en protéine HR23A endogène.

7. Procédé selon l'une quelconque des revendications 1 à 6, dans lequel ladite cellule est déficiente en protéine HR23B endogène.

8. Procédé selon l'une quelconque des revendications 1 à 7, dans lequel ladite cellule fait partie d'une lignée cellulaire.

9. Procédé selon la revendication 8, dans lequel ladite lignée cellulaire comprend une lignée cellulaire de fibroblastes embryonnaires de souris.

10. Procédé selon la revendication 8 ou 9, dans lequel les cellules de ladite lignée cellulaire sont déficientes en protéine HR23A endogène.

11. Procédé selon l'une quelconque des revendications 8 à 10, dans lequel les cellules de ladite lignée cellulaire sont déficientes en protéine HR23B endogène.

12. Procédé selon l'une quelconque des revendications 1 à 11, dans lequel ladite cellule est pourvue d'un acide nucléique codant la protéine HR23A humaine.

13. Procédé selon l'une quelconque des revendications 1 à 12, dans lequel ladite cellule est pourvue d'un acide nucléique codant la protéine HR23A murine.

14. Procédé selon l'une quelconque des revendications 1 à 13, dans lequel ladite cellule a été pourvue d'un acide nucléique codant la protéine HR23B humaine.

15. Procédé selon l'une quelconque des revendications 1 à 14, dans lequel ladite cellule a été pourvue d'un acide nucléique codant la protéine HR23B murine.

16. Procédé selon l'une quelconque des revendications 1 à 15, dans lequel ladite protéine du groupe C de *xeroderma pigmentosum* comprend une protéine humaine du groupe C de *xeroderma pigmentosum.*

17. Procédé selon l'une quelconque des revendications 1 à 16, dans lequel ladite protéine du groupe C de *xeroderma pigmentosum* ou une partie fonctionnelle, un dérivé et/ou un analogue de celle-ci, comprend un marqueur.

18. Procédé selon la revendication 17, dans lequel ledit marqueur comprend la protéine fluorescente verte ou la luciférase.

19. Procédé pour déterminer si un agent est capable d'inhiber, au moins en partie, un processus cellulaire, ledit processus aboutissant à une accumulation intranucléaire globale de protéine du groupe C de *xeroderma pigmentosum,* ou d'une partie fonctionnelle de celle-ci qui présente les mêmes propriétés que la protéine XPC, dans une cellule, le procédé comprenant les étapes consistant à:
- exposer au moins une cellule eucaryote audit agent, et
- déterminer si une accumulation intranucléaire globale de protéine du groupe C de *xeroderma pigmentosum,* ou d'une partie fonctionnelle, d'un dérivé et/ou d'un analogue de celle-ci qui présentent le même type de propriétés que la protéine XPC, se produit dans ladite cellule.

20. Procédé selon la revendication 19, dans lequel ladite cellule eucaryote comprend une lésion de l'ADN.

21. Procédé selon la revendication 19 ou 20, dans lequel ledit procédé comprend la protéolyse, la migration depuis le noyau vers le cytoplasme, la synthèse d'ARN, la maturation de l'ARN, le transport de l'ARN et/ou la traduction de l'ARN.

22. Procédé selon la revendication 21, dans lequel ladite protéolyse comprend une protéolyse protéasomale.

23. Procédé pour déterminer si une cellule comprend un système, au moins partiellement altéré, de réparation globale du génome par excision de nucléotides, comprenant les étapes consistant à :
- exposer ladite cellule à un agent capable d'induire une lésion de l'ADN, et
- déterminer si une accumulation intranucléaire globale de protéine du groupe C de *xeroderma pigmentosum,* ou d'une partie fonctionnelle de celle-ci qui présente les mêmes propriétés que la protéine XPC, se produit dans ladite cellule.

24. Procédé pour déterminer si un individu souffre, ou risque de souffrir, d'une maladie liée à un système, au moins partiellement altéré, de réparation globale du génome par excision de nucléotides, comprenant les étapes consistant à :
- exposer au moins une cellule ayant été obtenue sur ledit individu à un agent capable d'induire une lésion de l'ADN, et
- déterminer si une accumulation intranucléaire globale de protéine du groupe C de *xeroderma pigmentosum,* ou d'une partie fonctionnelle de celle-ci qui présente les mêmes propriétés que la protéine XPC, se produit dans le noyau de ladite cellule.

25. Procédé selon la revendication 23 ou 24, dans lequel ladite maladie comprend *xeroderma pigmentosum,* le syndrome de Cockayne et/ou la trichothiodystrophie.

26. Kit comprenant une cellule eucaryote qui est déficiente en protéine HR23A endogène et/ou en protéine HR23B endogène et un agent capable d'induire une lésion de l'ADN qui est un substrat pour une réparation globale du génome par excision de nucléotides et/ou par excision de bases.

27. Kit selon la revendication 26, comprenant en outre un système de détection pour détecter une variation du niveau de protéine du groupe C de *xeroderma pigmentosum* et/ou de 3-méthyladénine ADN-glycosylase, ou d'une partie fonctionnelle de celles-ci.
